(19) Europäisches Patentamt
European Patent Office
Office européen des brevets

(11) EP 3 590 514 A1

(12) EUROPEAN PATENT APPLICATION
published in accordance with Art. 153(4) EPC

(43) Date of publication:
**08.01.2020 Bulletin 2020/02**

(21) Application number: **18761226.2**

(22) Date of filing: **28.02.2018**

(51) Int Cl.:
***A61K 31/472** (2006.01)* ***A61J 1/05** (2006.01)*
***A61K 9/08** (2006.01)* ***A61K 31/4725** (2006.01)*
***A61K 47/04** (2006.01)* ***A61K 47/10** (2017.01)*
***A61P 27/02** (2006.01)* ***A61P 27/06** (2006.01)*
***B65D 1/00** (2006.01)* ***B65D 65/20** (2006.01)*

(86) International application number:
**PCT/JP2018/007581**

(87) International publication number:
**WO 2018/159700 (07.09.2018 Gazette 2018/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **28.02.2017 JP 2017037480**

(71) Applicant: **Kowa Company, Ltd.**
**Nagoya-shi, Aichi 460-8625 (JP)**

(72) Inventor: **YAMAKITA, Yoshihiro**
**Fuji-shi**
**Shizuoka 417-8650 (JP)**

(74) Representative: **Schiener, Jens**
**Wächtershäuser & Hartz**
**Patentanwaltspartnerschaft mbB**
**Weinstraße 8**
**80333 München (DE)**

# (54) MEDICINAL PREPARATION

(57) To establish a technique for stably formulating an isoquinolin-6-amino derivative as an ophthalmic agent or the like. A pharmaceutical preparation is provided including an aqueous composition containing a compound represented by the following formula (1)

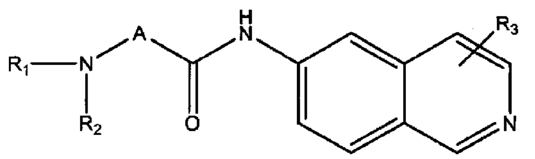
(1)

(in the formula, $R_1$ and $R_2$ each independently represent a hydrogen atom or a $C_1$-$C_4$ alkyl group, $R_3$ represents a hydrogen atom or a hydroxy group, and "A" represents-$CH(R_4)$- or -$CH_2$-$CH(R_4)$- (wherein $R_4$ represents an optionally substituted $C_6$-$C_{10}$ aryl group, or an optionally substituted 5 to 10-membered heteroaryl group), and the formula (1) also includes a tautomer thereof) or a salt thereof or a solvate thereof, the aqueous composition being housed in a package which blocks a light having a wavelength of from 320 to 380 nm.

EP 3 590 514 A1

**Description**

Technical Field

The present invention relates to a pharmaceutical preparation and the like.

Background Art

Heretofore, several isoquinolin-6-amino derivatives have been known to be useful in prevention and treatment of eye diseases such as ocular hypertension and glaucoma. Specifically, for example, a compound represented by the following structural formula:

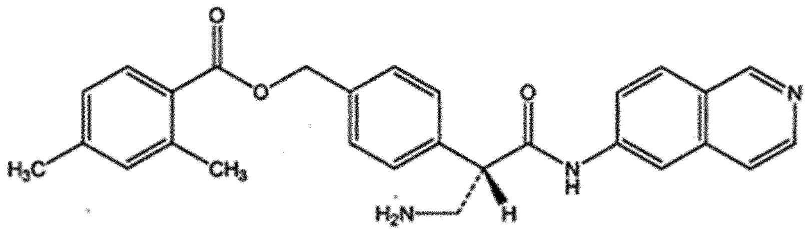

(Chemical Name: [4-[(2S)-3-amino-1-(isoquinolin-6-ylamino)-1-oxopropan-2-yl]phenyl]methyl 2,4-dimethylbenzoate), International Nonproprietary Name: netarsudil, hereinafter sometimes referred to as "netarsudil"), also known as AR-13324, has been reported to have pharmacological effects such as Rho kinase inhibitory effect and norepinephrine transporter inhibitory effect, and be useful in prevention and treatment of eye diseases such as ocular hypertension and glaucoma (for example, Patent Literature 1, and Non Patent Literatures 1 and 2).

Similarly, a compound represented by the following structural formula:

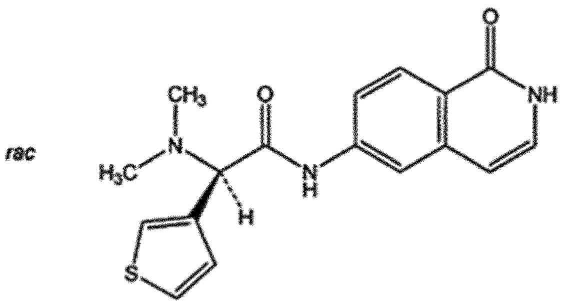

(Chemical Name: rac-(2R)-2-(dimethylamino)-N-(1-oxo-1,2-dihydroisoquinolin-6-yl)-2-(thiophen-3-yl)acetamide), International Nonproprietary Name: verosudil, hereinafter sometimes referred to as "verosudil"), also known as AR-12286, has been reported to have Rho kinase inhibitory effect and be useful in prevention and treatment of eye diseases such as ocular hypertension (for example, Patent Literature 2, and Non Patent Literatures 1 and 3).

Accordingly, it is very useful to establish a technique for stably formulating these isoquinolin-6-amino derivatives, for example, as an ophthalmic agent or the like.

Meanwhile, Patent Literature 3 states that a composition containing netarsudil or verosudil was prepared in a 150 mL plastic container. However, in the literature, each composition was prepared to use in pharmacological studies. The literature does not describe the stability of netarsudil and verosudil at all, neither describes the material and characteristics of the container at all.

Citation List

Patent Literature

Patent Literature 1: JP-A-2012-525386
Patent Literature 2: WO2009/091898

Patent Literature 3: JP-A-2016-515520

Non Patent Literature

Non Patent Literature 1: Bacharach J. et al., Ophthalmology 122(2) 302-7 (2015)
Non Patent Literature 2: Sturdivant JM. et al., Bioorg Med Chem Lett. 26(10) 2475-80 (2016)
Non Patent Literature 3: Williams RD. et al., Am. J. Ophthalmol. 152(5) 834-41 (2011)

Summary of Invention

Technical Problem

An object of the present invention is to establish a technique for stably formulating an isoquinolin-6-amino derivative such as netarsudil or verosudil as an ophthalmic agent or the like.

Solution to Problem

An ophthalmic agent or the like is usually a composition containing water (an aqueous composition). Thus, the present inventor prepared an aqueous composition comprising an isoquinolin-6-amino derivative such as netarsudil and housed the prepared composition in a container to check its storage property. However, unexpectedly, it was revealed that the content of isoquinolin-6-amino derivative is reduced with time due to exposure to light.

Therefore, the present inventor conducted further extensive studies to improve light stability of the isoquinolin-6-amino derivative in an aqueous composition. Consequently, the inventor has found that the content reduction of the isoquinolin-6-amino derivative in the aqueous composition is caused by irradiation of light of a certain wavelength, and that, by housing the aqueous composition comprising the isoquinolin-6-amino derivative in a package which blocks a light of the certain wavelength, a pharmaceutical preparation having improved light stability can be obtained, and has completed the present invention.

The present invention provides a pharmaceutical preparation comprising: an aqueous composition comprising a compound represented by the following formula (1)

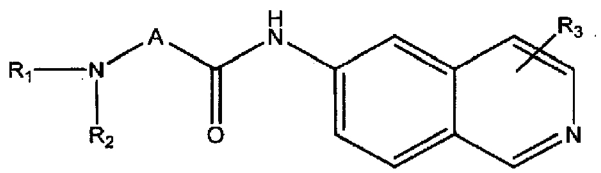

(1)

(in the formula, $R_1$ and $R_2$ each independently represent a hydrogen atom or a $C_1$-$C_4$ alkyl group,
$R_3$ represents a hydrogen atom or a hydroxy group, and
"A" represents -$CH(R_4)$- or -$CH_2$-$CH(R_4)$- (wherein $R_4$ represents an optionally substituted $C_6$-$C_{10}$ aryl group, or an optionally substituted 5 to 10-membered heteroaryl group), and
the formula (1) also includes a tautomer thereof) or a salt thereof or a solvate thereof, the aqueous composition being housed in a package which blocks a light having a wavelength of from 320 to 380 nm.

Advantageous Effects of Invention

According to the present invention, it is possible to improve the light stability of isoquinolin-6-amino derivatives exemplified by netarsudil in an aqueous composition.

Brief Description of Drawings

[Fig. 1] Fig. 1 is a graph showing the transmittance (%) of the light having a wavelength in the range of from 270 to 800 nm of an eye drop container formed of polypropylene into which a benzotriazole-based ultraviolet absorbing agent was kneaded, the container being used in Test Example 3.

[Fig. 2] Fig. 2 is a graph showing the transmittance (%) of the light having a wavelength in the range of from 270 to 800 nm of an eye drop container formed of polypropylene and having a shrink film blended with an ultraviolet scattering agent, the container being used in Test Example 4.

[Fig. 3] Fig. 3 is a graph showing the transmittance (%) of the light having a wavelength of from 270 to 800 nm of a bag for eye drop instillation formed of polyethylene blended with an ultraviolet absorbing agent, the bag being used in Test Example 5.

Description of Embodiments

In the present specification, "a compound represented by the formula (1):

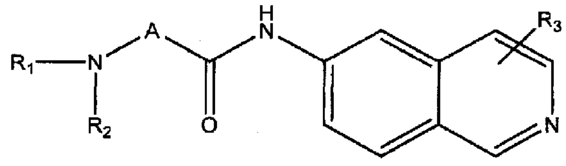

(1)

(in the formula, $R_1$ and $R_2$ each independently represent a hydrogen atom or a $C_1$-$C_4$ alkyl group,

$R_3$ represents a hydrogen atom or a hydroxy group, and

"A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and

the formula (1) also includes a tautomer thereof)

or a salt thereof or a solvate thereof" includes not only a compound represented by the formula (1) itself but also a salt or solvate thereof.

Examples of the salt of the compound represented by the formula (1) are not particularly limited as long as those are pharmaceutically acceptable salts. Specific examples of the salt include an inorganic acid salt such as a hydrochloride, a sulfate, a nitrate, a hydrofluoride, or a hydrobromide; an organic acid salt such as an acetate, a tartrate, a lactate, a citrate, a fumarate, a maleate, a succinate, a methanesulfonate, an ethanesulfonate, a benzenesulfonate, a toluenesulfonate, a naphthalenesulfonate, or a camphorsulfonate, and the hydrochloride and the methanesulfonate are preferred.

Examples of the solvate of the compound represented by the formula (1) or a salt thereof include a hydrate and an alcohol solvate.

Furthermore, when an asymmetric carbon exists in a chemical structure of the compound represented by the formula (1), various stereoisomers can exist, but the configuration of the compound represented by the formula (1) is not particularly limited, and the compound may be a single stereoisomer or a mixture of various stereoisomers in any proportions.

In the specification, when an asymmetric carbon exists in a chemical structure of the compound represented by various formulae, the compound represented by the formula encompasses all of a variety of single stereoisomers and mixtures of the stereoisomers in any proportions, unless otherwise specifies the configuration. Therefore, compounds represented by the formula for which the configuration is not especially specified may be a single stereoisomer or a mixture of various stereoisomers in any proportions.

The compound represented by the formula (1) includes not only a compound directly represented by the formula (1), but also a tautomer of the compound. Specifically, for example, when the formula (1) is the following formula (1a) in which $R_3$ is a hydroxy group, a tautomer thereof (formula (1b)) can exist, then the "compound represented by the formula (1)" includes not only a compound represented by the following formula (1a), but also a compound represented by the formula (1b).

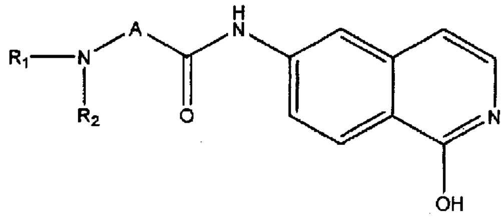

(1 a)

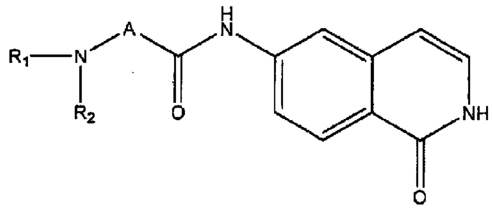

(1 b)

In the present specification, the "$C_1$-$C_4$ alkyl group" means a linear, branched or cyclic alkyl group having 1 to 4 carbon atoms. Specific examples of the "$C_1$-$C_4$ alkyl group" include a methyl group, an ethyl group, a n-propyl group, an isopropyl group, a cyclopropyl group, a n-butyl group, a t-butyl group, and an isobutyl group, and the methyl group is preferable.

In the present specification, the "$C_6$-$C_{10}$ aryl group" means an aryl group having 6 to 10 carbon atoms. Specific examples of the "$C_6$-$C_{10}$ aryl group" include a phenyl group and a naphthyl group, and the phenyl group is preferable.

In the present specification, the "5 to 10-membered heteroaryl group" means a 5 to 10-membered monocyclic, polycyclic or condensed cyclic aromatic heterocyclic group having 1 to 4 heteroatoms selected from the group consisting of an oxygen atom, a sulfur atom, and a nitrogen atom as a ring-constituting atom(s). Specific examples of the "5 to 10-membered heteroaryl group" include a furyl group, a thienyl group, a pyrrolyl group, an oxazolyl group, an isoxazolyl group, a thiazolyl group, an isothiazolyl group, an imidazolyl group, a pyrazolyl group, an oxadiazolyl group, a thiadiazolyl group, a triazolyl group, a tetrazolyl group, a pyridyl group, a pyrimidyl group, a pyrazinyl group, a pyridazinyl group, a benzofuranyl group, an isobenzofuranyl group, a benzothienyl group, an indolyl group, an isoindolyl group, an indazolyl group, a benzimidazolyl group, a benzoxazolyl group, a benzisoxazolyl group, a benzothiazolyl group, a benzisothiazolyl group, a benzoxadiazolyl group, a benzothiadiazolyl group, a benzotriazolyl group, a quinolyl group, an isoquinolyl group, a cinnolinyl group, a quinazolinyl group, a quinoxalinyl group, a phthalazinyl group, a naphthyridinyl group, a purinyl group, a pteridinyl group, a furopyridyl group, a thienopyridyl group, a pyrrolopyridyl group, an oxazolopyridyl group, a thiazolopyridyl group, and an imidazopyridyl group, and the thienyl group is preferable.

In the specification, examples of the "substituent" in the "optionally substituted $C_6$-$C_{10}$ aryl group" and "optionally substituted 5 to 10-membered heteroaryl group" specifically include a halogen atom and $-CH_2-OC(=O)-R_5$ (wherein, $R_5$ means a $C_6$-$C_{10}$ aryl group optionally having one to three $C_1$-$C_4$ alkyl group(s) as substituent(s) (e.g., 2,4-dimethylphenyl group), and a chlorine atom and a 2,4-dimethylphenyl carboxymethyl group are preferable.

In the specification, it is preferred that the "optionally substituted $C_6$-$C_{10}$ aryl group" is a 4-chlorophenyl group and a 4-(2,4-dimethylphenyl carboxymethyl)phenyl group.

In the specification, it is preferred that the "optionally substituted 5 to 10-membered heteroaryl group" is a 3-thienyl group.

When $R_3$ is a hydroxy group, the substitution position of $R_3$ is not limited as long as it is on the isoquinoline ring, but it is preferably position 1 of the isoquinoline ring.

In the specification, the "compound represented by the formula (1) or a salt thereof or a solvate thereof" is preferably a compound represented by the following formula (2):

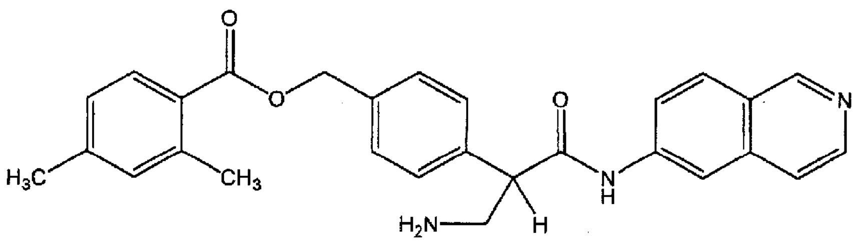

(2)

or a salt thereof or a solvate thereof; more preferably a compound represented by the following formula (3):

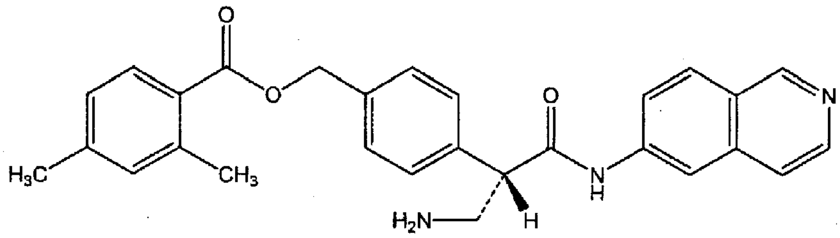

(3)

(netarsudil)(Chemical name: [4-[(2S)-3-amino-1-(isoquinolin-6-ylamino)-1-oxopropan-2-yl]phenyl]methyl 2,4-dimethylbenzoate), International Nonproprietary Name: netarsudil) or a salt thereof or a solvate thereof; and particularly preferably a compound represented by the following formula (4):

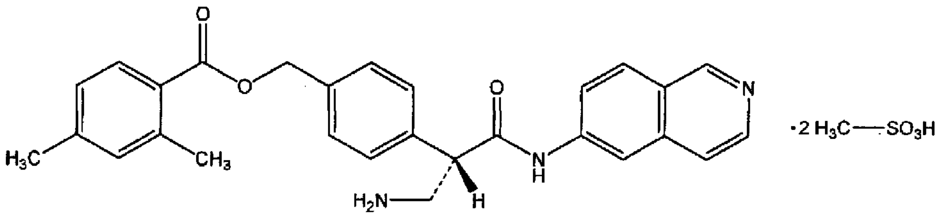

(4)

, which is a dimethanesulfonate of the compound represented by the formula (3), (chemical name: [4-[(2S)-3-amino-1-(isoquinolin-6-ylamino)-1-oxopropan-2-yl]phenyl]methyl 2,4-dimethylbenzoate dimethanesulfonate, hereinafter in the specification, it is sometimes referred to as "netarsudil dimesylate").

As another embodiment, the "compound represented by the formula (1) or a salt thereof or a solvate thereof" is preferably a compound represented by the following formula (5) or (5'):

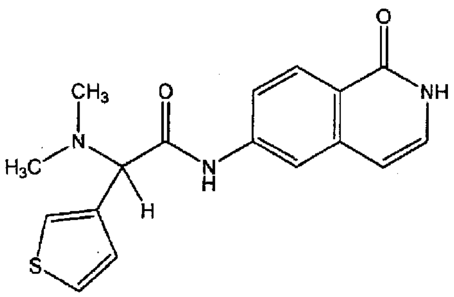

(5)

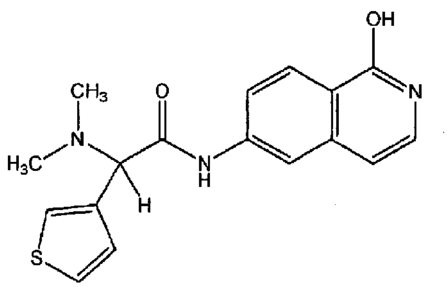

(5')

or a salt thereof or a solvate thereof; more preferably a compound represented by the following formula (6):

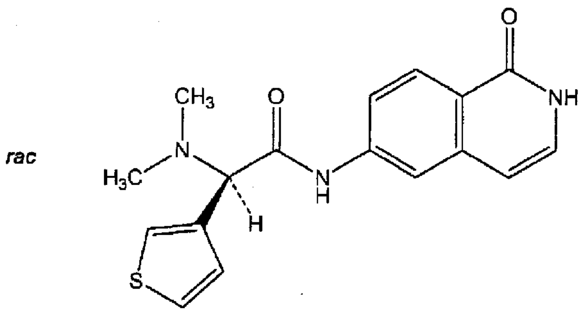

(6)

(verosudil) (Chemical name: rac-(2R)-2-(dimethylamino)-N-(1-oxo-1,2-dihydroisoquinolin-6-yl)-2-(thiophen-3-yl)acetamide), International Nonproprietary Name: verosudil), or a tautomer thereof or a salt of these or a solvate thereof; and particularly preferably a compound represented by the following formula (7):

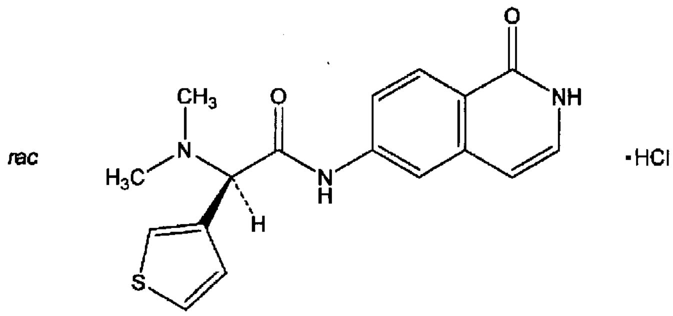

(7)

, which is a monohydrochloride of the compound represented by the formula (6), (chemical name: rac-(2R)-2-(dimethylamino)-N-(1-oxo-1,2-dihydroisoquinolin-6-yl)-2-(thiophen-3-yl)acetamide monohydrochloride, hereinafter in the specification, it is sometimes referred to as "verosudil monohydrochloride").

Further, as another embodiment, the "compound represented by the formula (1) or a salt thereof or a solvate thereof" is preferably a compound represented by the following formula (8):

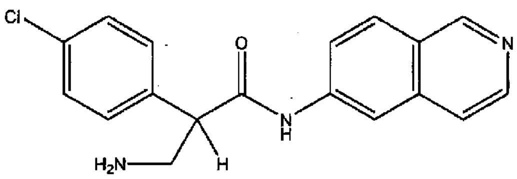

(8)

(3-amino-2-(4-chlorophenyl)-N-(isoquinolin-6-yl)propanamide) or a salt thereof or a solvate thereof.

The compound represented by the formula (1) or a salt thereof or a solvate thereof is known compounds, and they can be prepared by a known method. Specifically, for example, the compounds can be prepared with reference to the methods described in Patent Literatures 1 to 3 or Non Patent Literature 2. The contents of these literatures are incorporated herein by reference.

The compound represented by the formula (1) or a salt thereof or a solvate thereof is commercially available, and these commercial products may be used. Specific examples of the commercial products include netarsudil dimesylate (the compound represented by the formula (4)) manufactured by MedChemExpress and by Chemscene LLS; netarsudil monohydrochloride (monohydrochloride of the compound represented by the formula (3)) manufactured by Shanghai Biopharmaleader Co., Ltd.; and verosudil (a free form of the compound represented by the formula (6)) manufactured by MedKoo Biosciences, Inc.

The content of the compound represented by the formula (1) or a salt thereof or a solvate thereof in the aqueous composition is not particularly limited, and can be appropriately determined depending on the applicable disease and patient's sex, age, symptoms or the like, but, from the viewpoint of obtaining an excellent pharmacological action, the content is preferably from 0.0001 to 5 w/v%, more preferably from 0.001 to 3 w/v%, still more preferably from 0.005 to 2 w/v %, and particularly preferably from 0.01 to 1 w/v% of the compound represented by the formula (1) in terms of a

free form relative to the total volume of the aqueous composition.

Particularly, when netarsudil is used as the compound represented by the formula (1), the content is preferably from 0.0001 to 1 w/v%, more preferably from 0.001 to 0.5 w/v%, still more preferably from 0.005 to 0.1 w/v%, and particularly preferably from 0.01 to 0.04 w/v% of netarsudil in terms of a free form relative to the total volume of the aqueous composition, from the viewpoint of obtaining excellent pharmacological action.

Further, when verosudil is used as the compound represented by the formula (1), from the viewpoint of obtaining excellent pharmacological action, the content is preferably from 0.01 to 3.5 w/v%, more preferably from 0.1 to 2.5 w/v%, still more preferably from 0.2 to 1.5 w/v%, and particularly preferably from 0.3 to 0.8 w/v% of verosudil in terms of a free form relative to the total volume of the aqueous composition.

Furthermore, when the compound represented by the formula (8) is used as the compound represented by the formula (1), the content is preferably from 0.0001 to 3 w/v%, more preferably from 0.001 to 2 w/v%, still more preferably from 0.005 to 1 w/v%, and particularly preferably from 0.01 to 0.5 w/v% of the compound represented by the formula (8) in terms of a free form relative to the total volume of the aqueous composition, from the viewpoint of obtaining excellent pharmacological action.

In the present specification, the "aqueous composition" means a composition comprising at least water, and the property of the aqueous composition is not particularly limited as long as it can be housed in a package described below, and for example, the aqueous composition may be in the form of liquid (solution or suspension), semi-solid (ointment), and the like. Examples of the water in the composition include purified water, water for injection, and sterilized purified water.

The content of water in the aqueous composition is not particularly limited, but preferably 5 % by mass or more, more preferably 20 % by mass or more, still more preferably 50 % by mass or more, even more preferably 90 % by mass or more, and particularly preferably 90 to 99.99 % by mass.

The aqueous composition may further comprise, in addition to the compound represented by the formula (1) or a salt thereof or a solvate thereof, one or more components selected from the group consisting of an acid, a quaternary ammonium type surfactant, and a polyhydric alcohol.

In the specification, the "acid" which may be suitably blended to the aqueous composition is not particularly limited, and the acid may be an organic or inorganic acid. Specific examples of the "acid" include "borate", "phosphate", and "aliphatic carboxylate", and it is preferred to use one or more of these appropriately in combination, and particularly preferred to use the "borate" as the "acid".

In the present specification, the "borate" means one or more selected from the group consisting of boric acid and a salt thereof (e.g., an alkali metal salt such as a sodium salt or a potassium salt; an alkaline earth metal salt such as a calcium salt; and an ammonium salt) and a solvate thereof (e.g., a hydrate).

Specific examples of the borate include boric acid, ammonium borate, and borax. In the specification, it is preferred that the "borate" is boric acid.

All these borates are known compounds, and they may be prepared by a known method or their commercial products may be used.

In the present specification, the "phosphate" means one or more selected from the group consisting of phosphoric acid and a salt thereof (e.g., an alkali metal salt such as a sodium salt or a potassium salt; an alkaline earth metal salt such as a calcium salt; and an ammonium salt) and a solvate thereof (e.g., a hydrate).

Specific examples of the phosphate preferably include those listed in Pharmaceutical Excipients Dictionary 2016 (published by Yakuji Nippo) such as phosphoric acid, calcium monohydrogen phosphate, sodium monohydrogen phosphate heptahydrate, trisodium phosphate, dibasic calcium phosphate, dibasic calcium phosphate fine granulated, dibasic sodium phosphate, dibasic sodium phosphate heptahydrate, dibasic sodium phosphate dihydrate, disodium hydrogen phosphate dihydrate, dibasic potassium phosphate, monoabasic potassium phosphate, monobasic calcium phosphate hydrate, sodium dihydrogen phosphate dihydrate, sodium dihydrogen phosphate monohydrate, crystalline sodium dihydrogen phosphate dihydrate, calcium pyrophosphate, sodium pyrophosphate, anhydrous sodium pyrophosphate, disodium hydrogen phosphate anhydrous, anhydrous tribasic sodium phosphate, anhydrous dibasic calcium phosphate, anhydrous dibasic calcium phosphate fine granulated, sodium dihydrogen phosphate anhydrous, calcium polyphosphate, or sodium polyphosphate, and one or more selected from the group consisting of phosphoric acid, dibasic sodium phosphate, monobasic potassium phosphate, sodium dihydrogen phosphate dihydrate, sodium dihydrogen phosphate monohydrate, crystalline sodium dihydrogen phosphate dihydrate, disodium hydrogen phosphate anhydrous and sodium dihydrogen phosphate anhydrous are preferable.

All these phosphates are known compounds, so that they may be prepared by a known method or their commercial products may be used. Further, the phosphate in the form of a salt or a complex with another component may be used.

In the specification, the "aliphatic carboxylate" means one or more selected from the group consisting of aliphatic carboxylic acid and a salt thereof (e.g., an alkali metal salt such as a sodium salt or a potassium salt; an alkaline earth metal salt such as a calcium salt or a magnesium salt; and an ammonium salt) and a solvate thereof (e.g., a hydrate).

Here, the number of carbon atoms in the aliphatic carboxylate is not particularly limited, but preferably from 2 to 12, more preferably from 2 to 6, and particularly preferably from 2 to 4. Further, the carbon chain may be linear or branched, and may be saturated or unsaturated. Additionally, the number of carboxyl groups in the aliphatic carboxylate is not particularly limited, but preferably from 1 to 4, more preferably from 2 to 4, and particularly preferably from 2 to 3.

The "aliphatic carboxylate" may be a carboxylic acid having an amino group as a substituent (amino acid), and a carboxylic acid having a hydroxy group as a substituent (oxycarboxylic acid). In this case, the number of the substituent is preferably from 1 to 3, and particularly preferably from 1 to 2.

Specific examples of such aliphatic carboxylate include aspartic acid or a salt thereof or a solvate thereof such as sodium L-aspartate, potassium L-aspartate or magnesium L-aspartate; alanine or a salt thereof or a solvate thereof such as DL-alanine or L-alanine; arginine or a salt thereof or a solvate thereof such as L-arginine or L-arginine hydrochloride; epsilon-aminocaproic acid; edetic acid or a salt thereof or a solvate thereof such as calcium disodium edetate, sodium edetate hydrate, or tetrasodium edetate; citric acid or a salt thereof or a solvate thereof such as calcium citrate, citric acid hydrate, sodium citrate hydrate (trisodium citrate dihydrate), monobasic sodium citrate, dibasic sodium citrate, anhydrous citric acid, or sodium citrate anhydrous; glycine or a salt thereof or a solvate thereof; gluconic acid or a salt thereof or a solvate thereof such as gluconic acid, calcium gluconate hydrate, sodium gluconate, or magnesium gluconate; glutamine or a salt thereof or a solvate thereof; glutamic acid or a salt thereof or a solvate thereof such as L-glutamic acid, L-glutamic acid hydrochloride, potassium L-glutamate, or sodium L-glutamate; succinic acid or a salt thereof or a solvate thereof such as succinic acid, monosodium succinate, or disodium succinate hexahydrate; acetic acid or a salt thereof or a solvate thereof such as acetic acid, ammonium acetate, potassium acetate, calcium acetate, sodium acetate hydrate, glacial acetic acid, or anhydrous sodium acetate; cysteine or a salt thereof or a solvate thereof such as L-cystine, L-cysteine, or L-cysteine hydrochloride hydrate; tartaric acid or a salt thereof or a solvate thereof such as tartaric acid, D-tartaric acid, potassium hydrogen tartrate, sodium DL-tartrate, sodium L-tartrate, or sodium potassium tartrate; sorbic acid or a salt thereof or a solvate thereof such as sorbic acid or potassium sorbate; lactic acid or a salt thereof or a solvate thereof such as lactic acid, sodium lactate solution, calcium lactate hydrate or aluminum lactate; histidine or a salt thereof or a solvate thereof such as L-histidine or L-histidine hydrochloride hydrate; phenylalanine or a salt thereof or a solvate thereof; malonic acid or a salt thereof or a solvate thereof; methionine or a salt thereof or a solvate thereof such as DL-methionine or L-methionine; lysine or a salt thereof or a solvate thereof such as L-lysine or L-lysine hydrochloride; malic acid or a salt thereof or a solvate thereof such as malic acid, DL-malic acid or sodium DL-malate; and leucine or a salt thereof or a solvate thereof such as dl-leucine or L-leucine.

All these aliphatic carboxylic acids are known compounds, so that they may be prepared by a known method or their commercial products may be used. Further, the aliphatic carboxylate in the form of a salt or a complex with another component may be used.

The content of the acid in the aqueous composition is not particularly limited and can be appropriately determined, but it is preferably from 0.0001 to 4 w/v%, more preferably from 0.001 to 3 w/v%, and particularly preferably from 0.002 to 2.5 w/v% relative to the total volume of the aqueous composition. Particularly, in the case of using a borate as the acid, the content of the borate is not particularly limited and can be appropriately determined, but preferably from 0.003 to 2 w/v%, more preferably from 0.01 to 1 w/v%, and particularly preferably from 0.03 to 0.5 w/v% relative to the total volume of the aqueous composition.

The content mass ratio between the compound represented by the formula (1) or a salt thereof or a solvate thereof and the acid in the aqueous composition is not particularly limited, but the content of the acid is preferably 0.0003 to 200 parts by mass, more preferably 0.003 to 80 parts by mass, and particularly preferably 0.02 to 30 parts by mass relative to 1 part by mass of the compound represented by the formula (1) or a salt thereof or a solvate thereof in terms of a free form. Above all, in the case of using a borate as the acid, the content mass ratio of the borate is not particularly limited, but the content of the borate is preferably from 0.0005 to 150 parts by mass, more preferably from 0.005 to 75 parts by mass, and particularly preferably from 0.03 to 25 parts by mass relative to 1 part by mass of the compound represented by the formula (1) or a salt thereof or a solvate thereof in terms of a free form.

In particular, in the case of using netarsudil as the compound represented by the formula (1) and using a borate as the acid, the content of the borate is preferably from 0.5 to 100 parts by mass, more preferably from 1 to 50 parts by mass, and particularly preferably from 2 to 20 parts by mass relative to 1 part by mass of netarsudil or a salt thereof or a solvate thereof in terms of a free form.

Further, in the case of using verosudil as the compound represented by the formula (1) and using a borate as the acid, the content of the borate is preferably from 0.001 to 5 parts by mass, more preferably from 0.01 to 1 part by mass, and particularly preferably from 0.05 to 0.5 parts by mass relative to 1 part by mass of verosudil or a salt thereof or a solvate thereof in terms of a free form.

Furthermore, in the case of using the compound represented by the formula (8) as the compound represented by the formula (1) and using a borate as the acid, the content of the borate is preferably from 0.01 to 80 parts by mass, more preferably from 0.05 to 40 parts by mass, and particularly preferably from 0.1 to 20 parts by mass relative to 1 part by mass of the compound represented by the formula (8) or a salt thereof or a solvate thereof in terms of a free form.

In the specification, examples of the "quaternary ammonium type surfactant" which can be suitably blended in the aqueous composition include benzalkonium chloride, benzethonium chloride, and benzododecinium bromide, those are known as antiseptic. The quaternary ammonium type surfactant is preferably benzalkonium chloride or benzododecinium bromide, and particularly preferably benzalkonium chloride.

These quaternary ammonium type surfactants are known compounds, so that they may be prepared by a known method or their commercial products may be used.

The content of the quaternary ammonium type surfactant in the aqueous composition is not particularly limited and may be appropriately determined, but preferably from 0.00005 to 0.3 w/v%, more preferably from 0.00025 to 0.2 w/v%, and particularly preferably from 0.00025 to 0.15 w/v% relative to the total volume of the aqueous composition. Above all, in the case of using benzalkonium chloride as the quaternary ammonium type surfactant, the content of the benzalkonium chloride is preferably from 0.0001 to 0.3 w/v%, more preferably from 0.0005 to 0.2 w/v%, and particularly preferably from 0.0005 to 0.15 w/v% relative to the total volume of the aqueous composition. Further, in the case of using benzododecinium bromide as the quaternary ammonium type surfactant, the content of the benzododecinium bromide is preferably from 0.0001 to 0.15 w/v%, more preferably from 0.0005 to 0.05 w/v%, and particularly preferably from 0.0005 to 0.02 w/v% relative to the total volume of the aqueous composition.

The content mass ratio between the compound represented by the formula (1) or a salt thereof or a solvate thereof in the aqueous composition and the quaternary ammonium type surfactant is not particularly limited, but the content of the quaternary ammonium type surfactant is preferably from 0.0005 to 8 parts by mass, more preferably from 0.005 to 7 parts by mass, and particularly preferably from 0.007 to 6 parts by mass relative to 1 part by mass of the compound represented by the formula (1) or a salt thereof or a solvate thereof in terms of a free form.

In particular, in the case of using netarsudil as the compound represented by the formula (1) and using benzalkonium chloride as the quaternary ammonium type surfactant, the content of the benzalkonium chloride is preferably from 0.05 to 5 parts by mass, more preferably from 0.1 to 4 parts by mass, and particularly preferably from 0.5 to 3 parts by mass relative to 1 part by mass of netarsudil or a salt thereof or a solvate thereof in terms of a free form.

Further, in the case of using verosudil as the compound represented by the formula (1) and using benzalkonium chloride as the quaternary ammonium type surfactant, the content of the benzalkonium chloride is preferably from 0.001 to 1 part by mass, more preferably from 0.005 to 0.5 parts by mass, and particularly preferably from 0.01 to 0.3 parts by mass relative to 1 part by mass of verosudil or a salt thereof or a solvate thereof in terms of a free form.

Furthermore, in the case of using a compound represented by the formula (8) as the compound represented by the formula (1) and using benzalkonium chloride as the quaternary ammonium type surfactant, the content of the benzalkonium chloride is preferably from 0.05 to 5 parts by mass, more preferably from 0.1 to 4 parts by mass, and particularly preferably from 0.5 to 3 parts by mass relative to 1 part by mass of the compound represented by the formula (8) or a salt thereof or a solvate thereof in terms of a free form.

In the case of using netarsudil as the compound represented by the formula (1) and using benzododecinium bromide as the quaternary ammonium type surfactant, the content of the benzododecinium bromide is preferably from 0.07 to 5 parts by mass, more preferably from 0.2 to 4 parts by mass, and particularly preferably from 0.6 to 3 parts by mass relative to 1 part by mass of netarsudil or a salt thereof or a solvate thereof in terms of a free form.

Further, in the case of using verosudil as the compound represented by the formula (1) and using benzododecinium bromide as the quaternary ammonium type surfactant, the content of the benzododecinium bromide is preferably from 0.003 to 0.8 part by mass, more preferably from 0.007 to 0.4 parts by mass, and particularly preferably from 0.01 to 0.1 parts by mass relative to 1 part by mass of verosudil or a salt thereof or a solvate thereof in terms of a free form.

Furthermore, in the case of using a compound represented by the formula (8) as the compound represented by the formula (1) and using benzododecinium bromide as the quaternary ammonium type surfactant, the content of the benzododecinium bromide is preferably from 0.07 to 5 parts by mass, more preferably from 0.2 to 4 parts by mass, and particularly preferably from 0.4 to 3 parts by mass relative to 1 part by mass of a compound represented by the formula (8) or a salt thereof or a solvate thereof in terms of a free form.

In the specification, the "polyhydric alcohol" which can be suitably blended in the aqueous composition means an alcohol having two or more hydroxy groups in the same molecule, and examples of the polyhydric alcohol include glycerin; saccharide such as fructose or glucose; sugar alcohol such as sorbitol, mannitol or xylitol; trometamol; propylene glycol; and macrogol such as macrogol 400, macrogol 4000 or macrogol 6000. Among them, the polyhydric alcohol is preferably glycerin, sorbitol, propylene glycol, mannitol, or macrogol, and particularly preferably D-mannitol.

The polyhydric alcohols are known compounds, and they may be prepared by a known method or their commercial products may be used.

The content of the polyhydric alcohol in the aqueous composition is not particularly limited and can be appropriately determined, but preferably from 0.01 to 30 w/v%, more preferably from 0.1 to 20 w/v%, and particularly preferably from 0.5 to 5 w/v% relative to the total volume of the aqueous composition.

The content mass ratio between the compound represented by the formula (1) or a salt thereof or a solvate thereof and the polyhydric alcohol in the aqueous composition is not particularly limited, but the content of the polyhydric

alcohol is preferably from 0.01 to 2500 parts by mass, more preferably from 0.05 to 1500 parts by mass, and particularly preferably from 0.5 to 750 parts by mass relative to 1 part by mass of the compound represented by the formula (1) or a salt thereof or a solvate thereof in terms of a free form.

In particular, in the case of using netarsudil as the compound represented by the formula (1), the content of polyhydric alcohol is preferably from 10 to 2000 parts by mass, more preferably from 20 to 1000 parts by mass, and particularly preferably from 30 to 500 parts by mass relative to 1 part by mass of netarsudil or a salt thereof or a solvate thereof in terms of a free form.

Further, in the case of using verosudil as the compound represented by the formula (1), the content of polyhydric alcohol is preferably from 0.1 to 200 parts by mass, more preferably from 0.5 to 100 parts by mass, and particularly preferably from 1 to 50 parts by mass relative to 1 part by mass of verosudil or a salt thereof or a solvate thereof in terms of a free form.

Furthermore, in the case of using a compound represented by the formula (8) as the compound represented by the formula (1), the content of polyhydric alcohol is preferably from 10 to 2000 parts by mass, more preferably from 20 to 1000 parts by mass, and particularly preferably from 30 to 500 parts by mass relative to 1 part by mass of the compound represented by the formula (8) or a salt thereof or a solvate thereof in terms of a free form.

The aqueous composition may comprise one or more additives utilized in pharmaceutical products, quasi-drugs and the like, besides the components described above, depending on the dosage form. Examples of the additives include an inorganic salt, an isotonic agent, a chelating agent, a stabilizing agent, a pH adjusting agent, a preservative, an antioxidant, a thickening agent, a surfactant, a solubilizer, a suspending agent, a refreshing agent, a dispersing agent, a preserving agent, an oily base, an emulsion base, and a water-soluble base. The "acid", "quaternary ammonium type surfactant" or "polyhydric alcohol" described above may be used as the additive.

Specific examples of the additive include ascorbic acid, potassium aspartate, sodium bisulfite, alginic acid, sodium benzoate, benzyl benzoate, fennel oil, ethanol, an ethylene-vinyl acetate copolymer, potassium chloride, calcium chloride hydrate, sodium chloride, magnesium chloride, alkyldiaminoethylglycine hydrochloride solution, a carboxyvinyl polymer, dried sodium sulfite, dried sodium carbonate, d-camphor, dl-camphor, creatinine, chlorobutanol, geraniol, sodium chondroitin sulfate, titanium oxide, gellan gum, dibutylhydroxytoluene, potassium bromide, sodium hydroxide, polyoxyl 45 stearate, purified lanolin, taurine, sodium hydrogen carbonate, sodium carbonate hydrate, sodium thiosulfate hydrate, thimerosal, tyloxapol, sodium dehydroacetate, a concentrated mixed tocopherol, white petrolatum, peppermint water, peppermint oil, ethyl parahydroxybenzoate, butyl parahydroxybenzoate, propyl parahydroxybenzoate, methyl parahydroxybenzoate, sodium hyaluronate, human serum albumin, hydroxyethyl cellulose, hydroxypropyl cellulose, hypromellose, sodium pyrosulfite, phenyl ethyl alcohol, bergamot oil, benzyl alcohol, povidone, polyoxyethylene (200) polyoxypropylene glycol (70), sodium polystyrenesulfonate, polysorbate 80, polyoxyethylene hydrogenated castor oil 60, d-borneol, methanesulfonic acid, methyl cellulose, 1-menthol, monoethanolamine, aluminum monostearate, polyethylene glycol monostearate, eucalyptus oil, potassium iodide, oxyquinoline sulfate, liquid paraffin, borneol, and vaseline.

Among the above, for example, potassium chloride, calcium chloride hydrate, sodium chloride, magnesium chloride, sodium hydroxide, sodium hydrogen carbonate, sodium carbonate hydrate, hydroxyethyl cellulose, hydroxypropyl cellulose, hypromellose, povidone, polysorbate 80, polyoxyethylene hydrogenated castor oil, polyethylene glycol monostearate, methyl cellulose, monoethanolamine, sodium hyaluronate, or 1-menthol is preferred as the additive.

The aqueous composition may further comprise, in addition to the components described above, one or more other medicinal components, depending on the applicable disease or the like. Examples of the medicinal components include $\alpha$1 receptor blockers including bunazosin or a salt thereof or a solvate thereof such as bunazosin hydrochloride; $\alpha$2 receptor agonists including brimonidine or a salt thereof or a solvate thereof such as brimonidine tartrate, and apraclonidine or a salt thereof or a solvate thereof; $\beta$-blockers including carteolol or a salt thereof or a solvate thereof such as carteolol hydrochloride, nipradilol or a salt thereof or a solvate thereof, timolol or a salt thereof or a solvate thereof such as timolol maleate, betaxolol or a salt thereof or a solvate thereof such as betaxolol hydrochloride, levobunolol or a salt thereof or a solvate thereof such as levobunolol hydrochloride, befunolol or a salt thereof or a solvate thereof, and metipranolol or a salt thereof or a solvate thereof; carbonic anhydrase inhibitors including dorzolamide or a salt thereof or a solvate thereof such as dorzolamide hydrochloride, brinzolamide or a salt thereof or a solvate thereof, acetazolamide or a salt thereof or a solvate thereof, dichlorphenamide or a salt thereof or a solvate thereof, and methazolamide or a salt thereof or a solvate thereof; prostaglandins and their derivatives (e.g., prostaglandin F2$\alpha$ derivatives) including isopropyl unoprostone or a salt thereof or a solvate thereof, tafluprost or a salt thereof or a solvate thereof, travoprost or a salt thereof or a solvate thereof, bimatoprost or a salt thereof or a solvate thereof, latanoprost or a salt thereof or a solvate thereof, cloprostenol or a salt thereof or a solvate thereof, and fluprostenol or a salt thereof or a solvate thereof; Rho kinase inhibitors including Ripasudil or a salt thereof or a solvate thereof, Y-39983, and H-1129; sympathomimetic drugs including dipivefrine or a salt thereof or a solvate thereof such as dipivefrin hydrochloride, and epinephrine or a salt thereof or a solvate thereof such as epinephrine, epinephrine borate, or epinephrine hydrochloride; parasympathomimetic drugs including distigmine bromide or a salt thereof or a solvate thereof, pilocarpine or a salt

thereof or a solvate thereof such as pilocarpine, pilocarpine hydrochloride or pilocarpine nitrate, and carbachol or a salt thereof or a solvate thereof; calcium antagonists including lomerizine or a salt thereof or a solvate thereof such as lomerizine hydrochloride; and cholinesterase inhibitors including demecarium or a salt thereof or a solvate thereof, echothiophate or a salt thereof or a solvate thereof, and physostigmine or a salt thereof or a solvate thereof. These medicinal components can be blended singly or a combination of two or more.

As the other medicinal components, β-blockers are preferred, and especially timolol is preferred.

The aqueous composition may be one which does not contain the prostaglandin and any derivative thereof. For example, one embodiment of the aqueous composition is preferably other than the following <A-1> to <A-10>:

<A-1> a composition comprising (rac)-2-(dimethylamino)-N-(1-hydroxy-isoquinolin-6-yl)-2-(thiophen-3-yl)acetamide hydrochloride, travoprost, boric acid, D-mannitol, benzalkonium chloride, polyoxyl 40 stearate, polyethylene glycol 400, EDTA, and purified water.

<A-2> a composition comprising (rac)-2-(dimethylamino)-N-(1-hydroxy-isoquinolin-6-yl)-2-(thiophen-3-yl)acetamide hydrochloride, travoprost, boric acid, D-mannitol, benzalkonium chloride, Cremophor RH40, polyethylene glycol 400, EDTA, and purified water.

<A-3> a composition comprising (rac)-2-(dimethylamino)-N-(1-hydroxy-isoquinolin-6-yl)-2-(thiophen-3-yl)acetamide hydrochloride, travoprost, boric acid, D-mannitol, polyoxyl 40 stearate, polyethylene glycol 400, EDTA, and purified water.

<A-4> a composition comprising (rac)-2-(dimethylamino)-N-(1-hydroxy-isoquinolin-6-yl)-2-(thiophen-3-yl)acetamide hydrochloride, latanoprost, monobasic sodium phosphate, dibasic sodium phosphate, benzalkonium chloride, sodium chloride, EDTA, and purified water.

<A-5> a composition comprising (rac)-2-(dimethylamino)-N-(1-hydroxy-isoquinolin-6-yl)-2-(thiophen-3-yl)acetamide hydrochloride, latanoprost, boric acid, D-mannitol, benzalkonium chloride, EDTA, and purified water.

<A-6> a composition comprising (rac)-2-(dimethylamino)-N-(1-hydroxy-isoquinolin-6-yl)-2-(thiophen-3-yl)acetamide hydrochloride, bimatoprost, monobasic sodium phosphate, dibasic sodium phosphate, benzalkonium chloride, sodium chloride, EDTA, and purified water.

<A-7> a composition comprising (rac)-2-(dimethylamino)-N-(1-hydroxy-isoquinolin-6-yl)-2-(thiophen-3-yl)acetamide hydrochloride, bimatoprost, monobasic sodium phosphate, dibasic sodium phosphate, benzalkonium chloride, sodium chloride, EDTA, and purified water.

<A-8> a composition comprising (rac)-2-(dimethylamino)-N-(1-hydroxy-isoquinolin-6-yl)-2-(thiophen-3-yl)acetamide hydrochloride, bimatoprost, boric acid, D-mannitol, and purified water.

<A-9> a composition comprising (S)-4-(3-amino-1-(isoquinolin-6-ylamino)-1-oxopropan-2-yl)benzyl 2,4-dimethylbenzoate, travoprost, boric acid, D-mannitol, benzalkonium chloride, polyoxyl 40 stearate, polyethylene glycol 400, EDTA, and purified water.

<A-10> a composition comprising (S)-4-(3-amino-1-(isoquinolin-6-ylamino)-1-oxopropan-2-yl)benzyl 2,4-dimethylbenzoate, latanoprost, boric acid, D-mannitol, benzalkonium chloride, polyoxyl 40 stearate, polyethylene glycol 400, EDTA, and purified water.

The pH of the aqueous composition (at 25°C) is not particularly limited, but preferably from 3 to 9, more preferably from 3.5 to 8, still more preferably from 4 to 7, particularly preferably from 5 to 6. Further, the osmotic pressure ratio of the aqueous composition to physiological saline is not particularly limited, but preferably from 0.6 to 3, particularly preferably from 0.6 to 2.

In the present specification, the "package" means a package for directly or indirectly housing the aqueous composition. Among the package, the container which directly houses the aqueous composition (for example, an eye drop container or the like in which an aqueous composition is directly filled) is particularly referred to as "primary package". Also, among the package, the package which indirectly houses the aqueous composition (i.e., the package which houses the primary package: for example, a bag for eye drop instillation (a bag housing the eye drop container)) is particularly referred to as "secondary package". The package is sufficient if it is able to block a light of the wavelength range described below under the typically expected storage condition for the pharmaceutical preparation, and the package may have a sealing property or not.

The concept of the package encompasses any of "well-closed container", "tight container" and "hermetic container" which are defined in the Japanese Pharmacopoeia, Seventeenth Edition, General Notices.

When the pharmaceutical preparation includes the secondary package, it is sufficient if at least one of the primary package and the secondary package blocks a light of the wavelength range described below. From the viewpoint of suppressing the content reduction of the compound represented by the formula (1) or a salt thereof or a solvate thereof not only during the distribution or storage but also during the use, it is preferred that at least the primary package blocks the light of the wavelength range described below.

The form of the package is not particularly limited as long as it is capable of housing the aqueous composition,

and may be appropriately selected and set depending on the dosage form, the application of the pharmaceutical preparation, or whether the package is the primary package or the secondary package, or the like. Of the package in such a form, specific examples of the primary package include a container for injection, a container for inhalation, a container for spray, a bottle-shaped container, a tube-shaped container, an eye drop container, a nasal drop container, an ear drop container, a bag shaped container, and the like. Further, specific examples of the secondary package include a packaging bag (e.g., a bag for eye drop instillation), a box (e.g., a paper box), a bottle (e.g., a glass bottle), and a can (e.g., an aluminum can).

When the pharmaceutical preparation includes, as the package, both of the primary package and the secondary package, the primary package is preferably an eye drop container, and the secondary package is preferably a bag for eye drop instillation, from the viewpoint of advantageously utilizing the pharmacological action of the compound represented by the formula (1).

The material (raw material) of the package is not particularly limited, and it may be appropriately selected depending on the form of the package. Specific examples of the material include glass, plastic, cellulose, pulp, rubber, and metal.

The material of the primary package for directly housing the aqueous composition is, from the viewpoint of processability, squeezability, durability and the like, preferably plastic or the like.

The material of the secondary package is, from the viewpoint of processability, preferably plastic, cellulose, pulp, paper, or the like.

When the material of the package is plastic, the plastic is preferably a thermoplastic resin, which may be a synthetic resin or a natural resin. Specific examples of the resin include a polyolefin resin, a polyester resin, a polyphenylene ether resin, a polycarbonate resin, a polysulfone resin, a polyamide resin, a polyvinyl chloride resin, and a styrene resin. These resins are preferably used singly or in a combination of two or more, and a mixture thereof (polymer alloy) may also be used.

In one embodiment, the material of the primary package is preferably one or more selected from the group consisting of a polyester resin and a polyolefin resin. As specifically disclosed in Test Example 6 described later, it was revealed that while the content reduction of the compound represented by the formula (1) may occur after an aqueous composition comprising the compound housed in a primary package is stored for a long time at high temperature, the content reduction is relatively suppressed so that a pharmaceutical preparation having excellent storage stability is obtained, when the material of the primary package is a polyester resin or a polyolefin resin.

In such case, it is sufficient if at least a portion of the primary package in contact with the aqueous composition is formed of one or more selected from the group consisting of a polyester resin and a polyolefin resin, thus, a package having a polyester resin or polyolefin resin portion in contact with the aqueous composition, even having a layer of other material laminated on the outside of the portion, also corresponds to the primary package "formed of one or more kinds of resin selected from the group consisting of a polyester resin and a polyolefin resin". Further, as used herein, the phrase "formed of a polyester resin" means that the material comprises, at least in part, a polyester resin. For example, the one formed of a mixture of two or more kinds of resin (polymer alloy) of a polyester resin and one or more other kinds of resin also corresponds to the one "formed of a polyester resin". Similarly, the phrase "formed of a polyolefin resin" means that the material comprises, at least in part, a polyolefin resin. For example, the one formed of a mixture of two or more kinds of resin (polymer alloy) of a polyolefin resin and one or more other kinds of resin also correspond to the one "formed of a polyolefin resin".

Here, dicarboxylic acid and diol which constitute the polyester resin are not particularly limited, and examples of the dicarboxylic acid include phthalic acid, terephthalic acid, and 2,6-naphthalenedicarboxylic acid, and examples of the diol include ethylene glycol, 1,3-propanediol, 1,4-butanediol, 1,4-cyclohexanedimethanol, and bisphenol. The polyester resin may be a polymer composed of single kind of polyester unit or a polymer composed of plural kinds of polyester units. In the case of a polymer composed of plural kinds of polyester units, the polymerization mode is not particularly limited, and the copolymer may be a random copolyer or a block copolymer. Furthermore, the stereoregularity (tacticity) of the polymer is not particularly limited.

Specific examples of the polyester resin include homopolyester such as polyalkylene terephthalate (e.g., polyethylene terephthalate, polybutylene terephthalate), polyalkylene naphthalate (e.g., polyethylene naphthalate, polybutylene naphthalate), polycycloalkylene terephthalate (e.g., poly(1,4-cyclohexylene dimethylene terephthalate) or polyarylate (e.g., resin composed of bisphenol and phthalic acid); copolyester comprising units of the homopolyester as the main component; and a copolymer of the homopolyester. These resins can be used singly or in a combination of two or more. From the viewpoint of suppressing the content reduction of the compound represented by the formula (1) at the time of storage at high temperature, the polyester resin is preferably, polyethylene terephthalate.

Further, the polyolefin resin is not particularly limited, and it may be a polymer composed of single kind of monomer (homopolymer) or a copolymer composed of plural kinds of monomers (copolymer). In the case of a copolymer, the polymerization mode is not particularly limited, and the copolymer may be a random copolymer or a block copolymer. Furthermore, the stereoregularity (tacticity) of the copolymer is not particularly limited.

Specific examples of the polyolefin resin include polyethylene (more specifically, including, low density polyethylene (linear low density polyethylene), high density polyethylene, medium density polyethylene), polypropylene, cyclic polyolefin, poly(4-methylpentene), polytetrafluoroethylene, an ethylene-propylene copolymer, an ethylene-$\alpha$-olefin copolymer, an ethylene-acrylic acid copolymer, an ethylene-methacrylic acid copolymer, an ethylene-vinyl acetate copolymer, and an ethylene-ethyl acrylate copolymer. These resins can be used singly or in a combination of two or more. From the viewpoint of suppressing the content reduction of the compound represented by the formula (1) due to storage under high temperature, the polyolefin resin is preferably, polyethylene, polypropylene, or cyclic polyolefin, more preferably, low density polyethylene, medium density polyethylene, high density polyethylene, or polypropylene, and particularly preferably, low density polyethylene, high density polyethylene, or polypropylene.

The package used in the present invention is a package which blocks a light having a wavelength of from 320 to 380 nm.

The wavelength range of the light to be blocked by the package is sufficient if it is in the range of from 320 to 380 nm (preferably, from 320 to 395 nm) in view of normal storage environment of the pharmaceutical preparation. However, from the viewpoint of improving the light stability, the wavelength range of the light to be blocked is preferably from 300 to 380 nm, more preferably from 300 to 395 nm. Further, considering the use under storage conditions where it is likely to be exposed to wavelength of particularly below 300 nm, the wavelength range of the light to be blocked is preferably from 270 to 380 nm, particularly preferably from 270 to 395 nm.

In the present specification, the terms "blocking a light" of the aforesaid wavelength range means that the average value of the transmittance of the light of the aforesaid wavelength range is 40% or less. Accordingly, for example, the "package which blocks a light having a wavelength of from 320 to 380 nm" means a package of which the average value of the transmittance of the light having a wavelength of from 320 to 380 nm is 40% or less. Here, for the embodiment where the pharmaceutical preparation includes the secondary package, when the package of the pharmaceutical preparation can "block the light" of the aforesaid wavelength range at least as a result of the combination (superimposition) of the primary package and the secondary package, the package in the pharmaceutical preparation corresponds to the "package which blocks a light", even when the primary package or the secondary package alone cannot block the light.

From the viewpoint of further improving light stability, the average value of the transmittance of the light of the aforesaid wavelength range of the package is preferably 35% or less, more preferably 30% or less, still more preferably 25% or less, even more preferably 20% or less, still even more preferably 15% or less, furthermore preferably 10% or less, and particularly preferably 5% or less.

In the present specification, the measurement of an average value of the transmittance of the light in a certain wavelength range can be carried out by measuring the transmittance of the light of the package in air in the certain wavelength range at 5 nm intervals using a spectrophotometer, and then calculating the average value of the measured transmittance. Examples of the spectrophotometer include U-3900 (Hitachi High-Technologies Corporation).

As specifically described in Test Examples 3 and 4, the light stability of the compound represented by the formula (1) in the aqueous composition can be improved by housing the aqueous composition comprising the compound represented by the formula (1) or a salt thereof or a solvate thereof in the package which blocks a light described above.

Specifically in the case where the aqueous composition is irradiated with light so that the cumulative irradiation dose at a temperature of 25±5°C is 1.2 million lux·hr using a D65 fluorescent lamp as a light source, it is possible to make the residual rate of the compound represented by the formula (1) in the aqueous composition after irradiation be, for example, 80% or more (preferably 85% or more, more preferably 90% or more, particularly preferably 95% or more). The residual rate of the compound represented by the formula (1) in the aqueous composition can be obtained by measuring the concentrations of the compound in the aqueous composition before and after light irradiation, respectively, and calculating the residual rate by the following expression using the measured concentrations.

Residual rate (%) of the compound represented by the formula (1) = {(Concentration of the compound represented by the formula (1) in the aqueous composition after light irradiation)/(Concentration of the compound represented by the formula (1) in the aqueous composition before light irradiation)} × 100

Specific measures to block the light in the aforesaid wavelength range are not particularly limited, but examples

of the methods include a method using a substance which blocks a light of the aforesaid wavelength range. More specific examples of the method include:
a method involving adding a substance which blocks a light of the aforesaid wavelength range to a package (for example, a method involving adding a substance which blocks a light of the aforesaid wavelength range to glass or resin and then forming the obtained mixture into a container shape):

a method involving providing a member (for example, a film) comprising a substance which blocks a light of the aforesaid wavelength range on the surface (at least one surface of the inside and outside surfaces) of a package (for example, the method involving adding a substance which blocks a light of the aforesaid wavelength range to a resin and forming the obtained resin into a heat shrink film, and then winding the film on the outside surface of the container);
a method involving applying a substance which blocks a light of the aforesaid wavelength range to the surface (at least one surface of the inside and outside surfaces of a package) of a package; and
a method involving using a substance that blocks a light of the aforesaid wavelength range as the main material of the package (for example, a method involving using a container formed of mainly metal or cardboard).

The substance which blocks a light of the aforesaid wavelength range is not particularly limited, but from the viewpoint of allowing the inside of the package to be visible, preferably a substance which interferes with transmission of ultraviolet light, such as an ultraviolet absorbing agent or an ultraviolet scattering agent. Specific examples of the ultraviolet scattering agent include titanium oxide and zinc oxide. Examples of the ultraviolet absorbing agent include: benzotriazole-based ultraviolet absorbent such as 2-(2H-benzotriazol-2-yl)-p-cresol (e.g., Tinuvin P: BASF), 2-(2H-benzotriazol-2-yl)-4,6-bis(1-methyl-1-phenylethyl)phenol (e.g., Tinuvin 234: BASF), 2-(3,5-di-tert-butyl-2-hydroxyphenyl)benzotriazole (e.g., Tinuvin 320: BASF), 2-[5-chloro-(2H)-benzotriazol-2-yl]-4-methyl-6-t-butyl-phenol (e.g., Tinuvin 326: BASF), 2-(3,5-di-t-butyl-2-hydroxyphenyl)-5-chlorobenzotriazole (e.g., Tinuvin 327: BASF), 2-(2H-benzotriazol-2-yl)-4,6-di-tert-pentylphenol (e.g., Tinuvin PA328: BASF), 2-(2H-benzotriazol-2-yl)-4-(1,1,3,3-tetramethylbutyl)phenol (e.g., Tinuvin 329: BASF), 2,2'-methylenebis[6-(2H-benzotriazol-2-yl)-4-(1,1,3,3-tetramethylbutyl)phenol (e.g., Tinuvin 360: BASF), a reaction product of methyl 3-(3-(2H-benzotriazol-2-yl)-5-tert-butyl-4-hydroxyphenyl)propionate and polyethylene glycol 300 (e.g., Tinuvin 213: BASF), 2-(2H-benzotriazol-2-yl)-6-dodecyl-4-methylphenol (e.g., Tinuvin 571: BASF), 2-(2'-hydroxy-3',5'-di-t-amylphenyl)benzotriazole, 2-[2'-hydroxy-3'-(3",4",5",6"-tetrahydrophthalimidemethyl)-5'-methylphenyl]benzotriazole, and 2,2'-methylenebis[4-(1,1,3,3-tetramethylbutyl)-6-(2H-benzotriazol-2-yl)phenol]; cyanoacrylate-based ultraviolet absorbing agent such as 2,2-bis{[2-cyano-3,3-diphenylacryloyloxy]methyl}propane-1,3-diyl=bis(2-cyano-3,3-diphenylacrylate) (for example, Uvinul 3030 FF: BASF), ethyl 2-cyano-3,3-diphenylacrylate (e.g., Uvinul 3035: BASF), and 2-ethylhexyl 2-cyano-3,3-diphenylacrylate (e.g., Uvinul 3039: BASF) and others; triazine-based ultraviolet absorbing agent such as 2-(4,6-diphenyl-1,3,5-triazin-2-yl)-5-[(hexyl)oxy]-phenol (e.g., Tinuvin 1577 ED: BASF); benzophenone-based ultraviolet absorbing agent such as octabenzone (e.g., Chimassorb 81: BASF), 2,2'-dihydroxy-4,4'-dimethoxybenzophenone (e.g., Uvinul 3049: BASF), 2,2'-4,4'-tetrahydrobenzophenone (e.g., Uvinul 3050: BASF), oxybenzone, hydroxymethoxybenzophenone sulfonic acid, sodium hydroxymethoxybenzophenone sulfonate, dihydroxydimethoxybenzophenone, sodium dihydroxydimethoxybenzophenone disulfonate, dihydroxybenzophenone, and tetrahydroxybenzophenone; cinnamic acid-based ultraviolet absorbing agent such as methyl diisopropylcinnamate, cinoxate, glyceryl mono-2-ethylhexanoate diparamethoxycinnamate, a mixture of isopropyl paramethoxycinnamate and diisopropyl cinnamate, 2-ethylhexyl paramethoxycinnamate, and benzyl cinnamate; benzoic acid ester-based ultraviolet absorbing agent such as para-aminobenzoic acid, ethyl para-aminobenzoate, glyceryl para-aminobenzoate, amyl para-dimethylaminobenzoate, 2-ethylhexyl para-dimethylaminobenzoate, and ethyl 4-[N,N-di(2-hydroxypropyl)amino]benzoate; salicylic acid-based ultraviolet absorbing agent such as ethylene glycol salicylate, octyl salicylate, dipropylene glycol salicylate, phenyl salicylate, homomenthyl salicylate, and methyl salicylate; guaiazulene; 2-ethylhexyl dimethoxybenzylidene dioxoimidazolidine propionate; 2,4,6-tris[4-(2-ethylhexyloxycarbonyl)anilino]1,3,5-triazine; para-hydroxyanisole; 4-tert-butyl-4'-methoxydibenzoylmethane; phenylbenzimidazole sulfonate; and hexyl 2-(4-diethylamino-2-hydroxybenzoyl)benzoate. As a substance which blocks a light of the aforesaid wavelength range, these substances may be used singly or in a suitable combination of two or more so as to block the light of the aforesaid wavelength range. The substance which blocks a light of the aforesaid wavelength range is preferably titanium oxide and benzotriazole-based ultraviolet absorbing agent.

When the substance which blocks a light of the aforesaid wavelength range is added to the material or member of the package, the blending ratio is determined depending on the kinds of the substance, the material of the package or additional member, and the like, but, for example, it may be about from 0.001 to 50 % by mass, preferably from 0.002 to 25 % by mass, particularly preferably from 0.01 to 10 % by mass relative to the material or additional member of the package.

In the present specification, the "package which blocks a light" includes a package in which only a portion of the package blocks a light. In such a package, based on the inner surface of the package, the portion which blocks a

light is preferably 10% or more portions, more preferably 30% or more portions, still more preferably 50% or more portions, and particularly preferably 70% or more portions relative to the total area of the inner surface.

It is preferred that the inside of the package is visible (observable) to the naked eye or the like. When the inside is visible, there are some advantages that it becomes possible to examine the presence or absence of contamination or the like in the manufacturing process of the pharmaceutical preparation; and that the user of the pharmaceutical preparation can check the remained content (aqueous composition), and the like.

In the present specification, the "inside is visible" means a state that the inside of the package is visible from at least a portion of the outer surface of the package. (For example, an eye drop container, which usually has a nearly cylindrical shape, having a bottom surface allowing the inside of the container to be visible corresponds to the container whose "inside is visible", even when the inside of the container is not visible from the side surface owing to a shrink film or the like.)

The visibility is sufficient if the package has a transparency of no less than a certain level, and specifically, for example, it is sufficient that the average value of the transmittance of the light in the visible light region (450 to 750 nm) is around 30% or more (more suitably around 40% or more, and particularly suitably around 50% or more), but the visibility is not limited thereto.

Preferred specific embodiments of the package include the following 1) to 4):

1) a primary package is a package (more preferably a package whose inside is visible) which is a container (preferably an eye drop container) (formed of plastic; more preferably formed of a polyolefin resin or a polyester resin) kneaded with a substance which interferes with transmission of ultraviolet light (more preferably one or more substances selected from the group consisting of an ultraviolet scattering agent and an ultraviolet absorbing agent; particularly preferably one or more substances selected from the group consisting of zinc oxide, titanium oxide and a benzotriazole-based ultraviolet absorbing agent);

2) a primary package is a package (more preferably a package whose inside is visible) which is a container (preferably an eye drop container) (preferably formed of plastic; more preferably formed of a polyolefin resin or a polyester resin) having a member (preferably a heat-shrinkable film (a shrink film)) kneaded with a substance which interferes with transmission of ultraviolet light (more preferably one or more substances selected from the group consisting of an ultraviolet scattering agent and an ultraviolet absorbing agent; particularly preferably one or more substances selected from the group consisting of zinc oxide, titanium oxide and a benzotriazole-based ultraviolet absorbing agent), the member being wound around on a side surface of the container.

3) a secondary package is a package (preferably a package whose inside is visible) which is a bag (preferably a bag for eye drop instillation) (preferably formed of plastic; more preferably formed of a polyolefin resin or a polyester resin) kneaded with a substance which interferes with transmission of ultraviolet light (preferably one or more substances selected from the group consisting of an ultraviolet scattering agent and an ultraviolet absorbing agent; more preferably one or more substances selected from the group consisting of zinc oxide, titanium oxide and a benzotriazole-based ultraviolet absorbing agent);

4) a secondary package is a package which is a box formed of paper which houses a container (preferably an eye drop container).

The means for housing the aqueous composition in the package is not particularly limited and it can be performed by filling or the like with an ordinary method depending on the form of the package or the like.

The pharmaceutical preparation or aqueous composition can be formulated in various dosage forms according to a known method described in the Japanese Pharmacopoeia, Seventeenth Edition, General Rules for Preparation or the like. Examples of the dosage form include injection, inhalation liquid, eye drop, eye ointment, ear drop, nasal drop liquid, enema formulation, topical liquid, spray, ointment, gel, oral liquid, and syrup. From the viewpoint of advantageously utilizing the pharmacological actions of the compound represented by the formula (1), the dosage form is preferably, a dosage form for eye disease, specifically, eye drop or eye ointment, and particularly preferably, eye drop.

The applicable disease of the pharmaceutical preparation is not particularly limited, and it is appropriately selected depending on the pharmacological actions of the compound represented by the formula (1) or the like.

Specifically, for example, the pharmaceutical preparation can be used as a prevention or treatment agent for ocular hypertension and glaucoma, based on Rho kinase inhibitory activity, norepinephrine transporter inhibitory activity, and intraocular pressure-decreasing activity of the compound represented by the formula (1). Detailed examples of the glaucoma include primary open-angle glaucoma, normal tension glaucoma, hypersecretion glaucoma, acute angle closure glaucoma, chronic angle closure glaucoma, plateau iris syndrome, mixed-type glaucoma, steroid glaucoma, glaucoma capsulare, pigment glaucoma, amyloid glaucoma, angiogenesis glaucoma, and malignant glaucoma.

In the case of using the aqueous composition or pharmaceutical preparation as a prevention or treatment agent for eye disease (appropriately, a diseases selected from the group of ocular hypertension and glaucoma), the aqueous composition or pharmaceutical preparation may be administered, for example, about 1 to 3 times a day in a suitable dose.

Hereinafter, examples of embodiments or aspects of the present invention are disclosed below; however, the present invention is not limited thereto.

[1] A pharmaceutical preparation comprising:
an aqueous composition comprising a compound represented by the following formula (1)

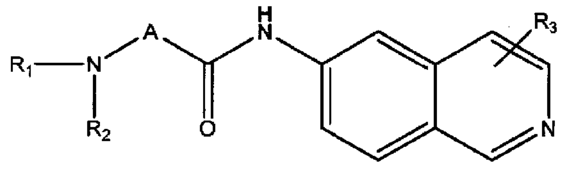

(1)

(in the formula, $R_1$ and $R_2$ each independently represent a hydrogen atom or a $C_1$-$C_4$ alkyl group, $R_3$ represents a hydrogen atom or a hydroxy group, and "A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and
the formula (1) also includes a tautomer thereof)
or a salt thereof or a solvate thereof, the aqueous composition being housed in a package which blocks a light having a wavelength of from 320 to 380 nm.

[2] The pharmaceutical preparation according to [1], wherein the compound represented by the formula (1) is a compound represented by the following formula (2), (5), (5') or (8) :

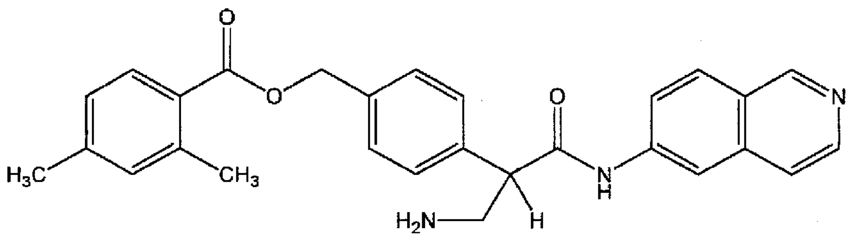

(2) ;

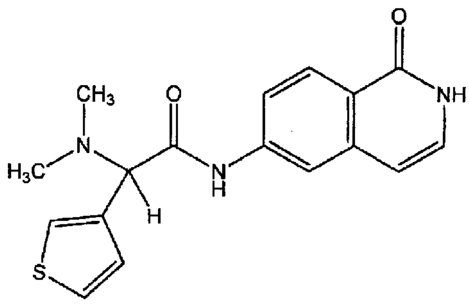

(5) ;

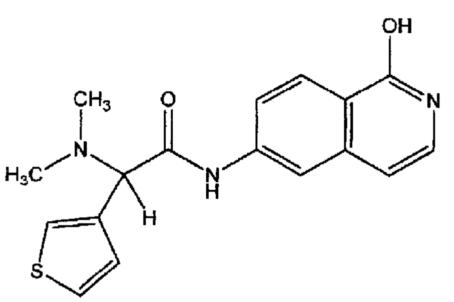

(5') ;

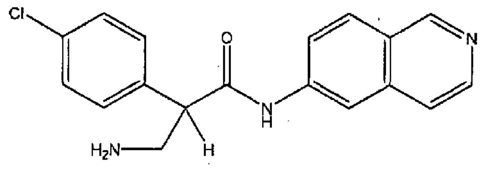

(8)

.

[3] The pharmaceutical preparation according to [1], wherein the compound represented by the formula (1) is a compound represented by the following formula (3) or (6):

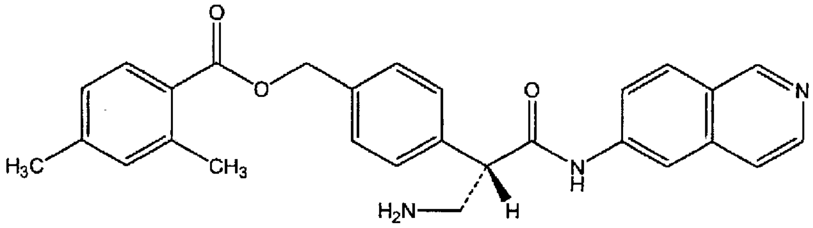

(3) ;

rac

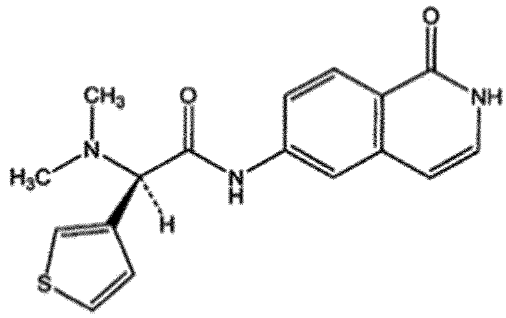

(6)

.

[4] The pharmaceutical preparation according to [1], wherein the compound represented by the formula (1) is a compound represented by the following formula (4) or (7):

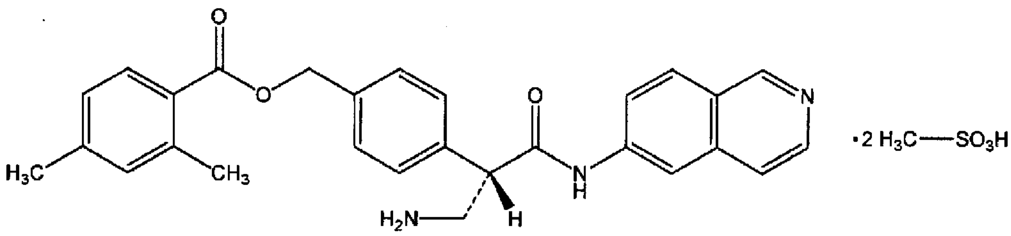

(4) ;

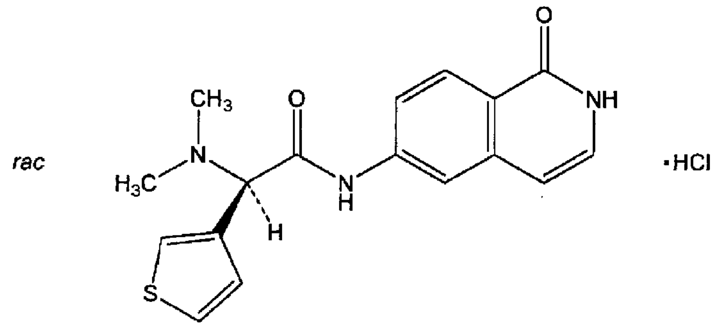

(7)

.

[5] The pharmaceutical preparation according to any one of [1] to [4], wherein the package is a package which blocks a light having a wavelength of from 320 to 395 nm (preferably from 320 to 395 nm).
[6] The pharmaceutical preparation according to any one of [1] to [5], wherein the package is a package which blocks a light having a wavelength of from 300 to 380 nm (preferably from 300 to 395 nm).
[7] The pharmaceutical preparation according to any one of [1] to [5], wherein the package is a package which blocks a light having a wavelength of from 270 to 380 nm (preferably from 270 to 395 nm).
[8] The pharmaceutical preparation according to any one of [1] to [7], wherein a residual rate of the compound represented by the formula (1) in the aqueous composition after irradiation is 80% or more (preferably 85% or more, more preferably 90% or more, still more preferably 95% or more, and particularly preferably 97% or more), when the aqueous composition is irradiated with a light so that a cumulative irradiation dose at a temperature of 25±5°C is 1.2 million lux·hr using a D65 fluorescent lamp as a light source.
[9] The pharmaceutical preparation according to any one of [1] to [8], wherein the inside of the package is visible.
[10] The pharmaceutical preparation according to any one of [1] to [9], wherein the package includes a primary package, and the primary package is:

a package (preferably a package whose inside is visible) which is a container (preferably an eye drop container) (preferably formed of plastic; more preferably formed of a polyolefin resin or a polyester resin) comprising a substance which interferes with transmission of ultraviolet light (preferably one or more substances selected from the group consisting of an ultraviolet scattering agent and an ultraviolet absorbing agent; more preferably one or more substances selected from the group consisting of zinc oxide, titanium oxide and a benzotriazole-based ultraviolet absorbing agent); or
a package (preferably a package whose inside is visible) which is a container (preferably an eye drop container) (preferably formed of plastic; more preferably formed of a polyolefin resin or a polyester resin) having a member (preferably a heat-shrinkable film (a shrink film)) comprising a substance which interferes with transmission of ultraviolet light (preferably one or more substances selected from the group consisting of an ultraviolet scattering agent and an ultraviolet absorbing agent; more preferably one or more substances selected from the group consisting of zinc oxide, titanium oxide and a benzotriazole-based ultraviolet absorbing agent).

[11] The pharmaceutical preparation according to any one of [1] to [10], wherein the package includes, as a secondary package, at least one selected from the group consisting of
a package (preferably a package whose inside is visible) which is a bag (preferably a bag for eye drop instillation) (preferably formed of plastic; more preferably formed of a polyolefin resin or a polyester resin) comprising a substance which interferes with transmission of ultraviolet light (preferably one or more substances selected from the group consisting of an ultraviolet scattering agent and an ultraviolet absorbing agent; more preferably one or more substances selected from the group consisting of zinc oxide, titanium oxide and a benzotriazole-based ultraviolet absorbing agent); and
a box formed of paper.
[12] The pharmaceutical preparation according to any one of [1] to [11], wherein the package includes a primary package, and the primary package is a container formed of a polyolefin resin.
[13] The pharmaceutical preparation according to any one of [1] to [11], wherein the package includes a primary package, and the primary package is a container formed of a polyester resin.
[14] A method for improving light stability of a compound represented by the following formula (1) or a salt thereof or a solvate thereof in an aqueous composition, comprising a step of housing the aqueous composition comprising the compound represented by the formula (1) or a salt thereof or a solvate thereof in a package which blocks a light having a wavelength of from 320 to 380 nm.

[15] The method according to [14], wherein the compound represented by the formula (1) is a compound represented by the formula (2), (5), (5') or (8).
[16] The method according to [14], wherein the compound represented by the formula (1) is a compound represented by the formula (3) or (6).
[17] The method according to [14], wherein the compound represented by the formula (1) is a compound represented by the formula (4) or (7).
[18] The method according to any one of [14] to [17], wherein the package is a package which blocks a light having a wavelength of from 320 to 395 nm.
[19] The method according to any one of [14] to [17], wherein the package is a package which blocks a light having a wavelength of from 300 to 380 nm (preferably from 300 to 395 nm).
[20] The method according to any one of [14] to [17], wherein the package is a package which blocks a light having a wavelength of from 270 to 380 nm (preferably from 270 to 395 nm).
[21] The method according to any one of [14] to [20], wherein the residual rate of the compound represented by the formula (1) in the aqueous composition after irradiation is 80% or more (preferably 85% or more, more preferably 90% or more, still more preferably 95% or more, and particularly preferably 97% or more), when the aqueous composition is irradiated with a light so that a cumulative irradiation dose at a temperature of 25±5°C is 1.2 million lux·hr using a D65 fluorescent lamp as a light source.
[22] The method according to any one of [14] to [21], wherein the inside of the package is visible.
[23] The method according to any one of [14] to [22], wherein the package includes a primary package, and the primary package is:

a package (preferably a package whose inside is visible) which is a container (preferably an eye drop container) (preferably formed of plastic; more preferably formed of a polyolefin resin or a polyester resin) comprising a substance which interferes with transmission of ultraviolet light (preferably one or more substances selected from the group consisting of an ultraviolet scattering agent and an ultraviolet absorbing agent; more preferably one or more substances selected from the group consisting of zinc oxide, titanium oxide and a benzotriazole-based ultraviolet absorbing agent); or
a package (preferably a package whose inside is visible) which is a container (preferably an eye drop container) (preferably formed of plastic; more preferably formed of a polyolefin resin or a polyester resin) having a member (preferably a heat-shrinkable film (a shrink film)) comprising a substance which interferes with transmission of ultraviolet light (preferably one or more substances selected from the group consisting of an ultraviolet scattering agent and an ultraviolet absorbing agent; more preferably one or more substances selected from the group consisting of zinc oxide, titanium oxide and a benzotriazole-based ultraviolet absorbing agent).

[24] The method according to any one of [14] to [23], wherein the package includes, as a secondary package, at least one selected from the group consisting of
a package (preferably a package whose inside is visible) which is a bag (preferably a bag for eye drop instillation) (preferably formed of plastic; more preferably formed of a polyolefin resin or a polyester resin) comprising a substance which interferes with transmission of ultraviolet light (preferably one or more substances selected from the group consisting of an ultraviolet scattering agent and an ultraviolet absorbing agent; more preferably one or more substances selected from the group consisting of zinc oxide, titanium oxide and a benzotriazole-based ultraviolet absorbing agent); and
a box formed of paper.
[25] The method according to any one of [14] to [24], wherein the package includes a primary package, and the primary package is a container formed of a polyolefin resin.
[26] The method according to any one of [14] to [24], wherein the package includes a primary package, and the primary package is a container formed of a polyester resin.

## Examples

Next, the present invention is further described by way of Examples, but the present invention is not limited to these Examples.
In the following Test Examples, the measurements of netarsudil using HPLC were performed using an ODS column as the column, 0.01 mol/L phosphate buffer solution and acetonitrile as the mobile phase, and a UV absorptiometer as the detector (wavelength: 254 nm), respectively.

[Test Example 1] Light Stability Test Part 1

The light stability of netarsudil blended in an aqueous composition was evaluated by examining the presence or absence of the content reduction of netarsudil due to exposure to light.

In other words, an aqueous composition having the formulation shown in Table 1 was prepared, and the prepared composition was housed in a transparent eye drop container formed of polypropylene. Then, the pharmaceutical preparation was irradiated using a light stability test device (LT-120A: Nagano Science Co., Ltd.) with a light of 4000 lux under 25°C using a D65 fluorescent lamp as a light source for 300 hours so that cumulative irradiation dose became 1.2 million lux·hr.

To examine the presence or absence of the content reduction of netarsudil due to exposure to light, the concentrations of netarsudil in the aqueous composition before and after the exposure to light were measured. The concentration of netarsudil in the aqueous composition was calculated by measuring the ratio of the peak area of the aqueous composition relative to the peak area of a netarsudil solution of a known concentration, using HPLC.

Then, based on the calculated concentration of netarsudil in the aqueous composition, the residual rate (%) of netarsudil was assessed using the following expression:

$$\text{Residual rate (\%)} = [(\text{Concentration of netarsudil in the aqueous composition after exposure to light})/(\text{Concentration of netarsudil in the aqueous composition before exposure to light})] \times 100$$

The results are shown in Table 2.

[Table 1]

| Component | Amount (per 100 mL) |
|---|---|
| Netarsudil dimesylate | 0.02848 g (0.02 g in terms of a free form) |
| Boric acid | 0.05 g |
| D-mannitol | 4.7 g |
| Benzalkonium chloride | 0.015 g |
| Sodium hydroxide | q.s. (pH 5.0) |
| Purified water | Balance |

[Table 2]

| | Cumulative irradiation dose: 1.2 million lux·hr |
|---|---|
| Residual rate (%) | 66.3 |

As shown in the results indicated in Table 2, it was revealed that the content of netarsudil in the aqueous composition decreased due to exposure to light. A D65 fluorescent lamp which irradiates a light having a wavelength of 300 nm or more was used as the light source.

From the test results above, it was revealed that the compound represented by the formula (1) exemplified by netarsudil or a salt thereof or a solvate thereof in the aqueous composition is unstable to light.

[Test Example 2] Light Stability Test Part 2

To identify the wavelengths of the light causing the content reduction of netarsudil due to exposure to light in the aqueous composition which was observed in Test Example 1, the light having the wavelengths of a certain range were irradiated, and the presence or absence of the content reduction due to exposure to light was evaluated.

In other words, the aqueous composition having the formulation shown in Table 1 was prepared, and the

prepared composition was housed in a transparent container formed of glass to obtain a pharmaceutical preparation. Then, using spectral radiation device having an optical filter (spectroscopy unit: HSU-100S, Light Source: MAX-302FBD, both from Asahi Spectra Co., Ltd.), the lights having a wavelength of approximately 270 to 335 nm, 320 to 395 nm, 380 to 410 nm, 430 to 475 nm, 470 to 535 nm or 350 to 800 nm each were irradiated on the pharmaceutical preparation under 25°C. The radiation energy of light at every wavelength was set to approximately 200 W·h/$m^2$.

To examine the presence or absence of the content reduction of netarsudil due to exposure to light, the residual rate (%) of netarsudil was assessed in the same method as in Test Example 1.

The results are shown in Table 3.

[Table 3]

| | Irradiation wavelength (nm) | | | | | |
|---|---|---|---|---|---|---|
| | 270-335 | 320-395 | 380-410 | 430-475 | 470-535 | 350-800 |
| Residual rate (%) | 83.7 | 77.8 | 95.1 | 96.6 | 96.1 | 98.5 |

As shown in the results in Table 3, the content reduction was scarcely observed by irradiation of a light having a wavelength of 380 nm or more, while significant content reduction was observed by irradiation of lights having wavelengths shorter than 380 nm, particularly 320 to 380 nm.

One of the tests to confirm the storage stability of pharmaceutical products is a light stability test, and the guideline of this test defines that D65 light source should be used as the light source. D65 light source is the internationally recognized standard for outdoor daylight as defined in ISO 10977 (1993), and prescribed with the value of the spectral distribution of wavelength of 300 to 830 nm. From these, it is believed that, as to the stability in the typically expected storage condition for pharmaceutical products, it is sufficient if the light having a wavelength of 300 nm or more is considered.

From the above, it was concluded that, for ensuring the light stability of aqueous composition comprising the compound represented by the formula (1) exemplified by netarsudil or a salt thereof or a solvate thereof, it is important to block the light, especially, of wavelength of from 320 to 380 nm.

[Test Example 3] Light Stability Test Part 3

Based on the results obtained in Test Example 2, in an attempt to obtain stabilization, the aqueous composition comprising netarsudil was housed in a container blended with an ultraviolet absorbing agent.

In other words, the aqueous composition having formulation shown in Table 1 was prepared, and the obtained aqueous composition was housed in an eye drop container (primary package) formed of polypropylene blended with an ultraviolet absorbing agent described below to obtain a pharmaceutical preparation. Then, the obtained pharmaceutical preparation was irradiated using a light stability test equipment (LT-120A: Nagano Science Co., Ltd.) with the light of 4000 lux under 25°C using a D65 fluorescent lamp as a light source for 300 hours so that cumulative irradiation dose becomes 1.2 million lux·hr.

To examine the presence or absence of the content reduction of netarsudil due to exposure to light, the residual rate (%) of netarsudil was assessed in the same method as in Test Example 1.

<Container Formed of Polypropylene Blended with Ultraviolet Absorbing Agent>

The eye drop container formed of polypropylene blended with benzotriazole-based ultraviolet absorbing agent was used. The average value of the transmittance of the light of the container was 18.6% in 320 to 380 nm (18.2% on average in 300 to 380 nm; 21.5% on average in 270 to 380 nm; 23.7% on average in 320 to 395 nm; 22.3% on average in 300 to 395 nm; and 24.3% on average in 270 to 395 nm).

The container appearance was nearly transparent and the content inside was visible (for example, the transmittance of the light in the visible light region (450 to 750 nm) was more than 75 % in all wavelengths and 81.8% on average.

The transmittance of the light was measured at 5 nm intervals using a spectrophotometer (U-3900: Hitachi High-Technologies Corporation) after the container was cut into a plate-like piece and set in an optical path so that light enters the piece nearly vertically. The average value of the transmittance of the light was calculated as an average value of the measured transmittance of the light in a predetermined wavelength range at 5 nm intervals.

In addition, a graph showing the relationship as to this container between the wavelength and transmittance (%) of the light in the range of 270 to 800 nm was shown in Fig. 1.

The results are shown in Table 4. For comparison, the test result of using the eye drop container formed of polypropylene not comprising the ultraviolet absorbing agent (average value of the transmittance of the light of 320 to

380 nm: 68.4%) (the result of Test Example 1) is also shown in a row.

[Table 4]

| | Properties of package | Cumulative irradiation dose: 1.2 million lux·hr |
|---|---|---|
| Residual rate (%) | Container (primary package) formed of polypropylene blended with ultraviolet absorbing agent Average value of transmittance of light of 320 to 380 nm: 18.6% | 96.0 |
| | Container (primary package) formed of polypropylene not comprising ultraviolet absorbing agent Average value of transmittance of light of 320 to 380 nm: 68.4% | 66.3 |

As shown in the results indicated in Table 4, it was revealed that, by housing the aqueous composition comprising netarsudil in a container (primary package) of which the average value of the transmittance of the light of 320 to 380 nm is 18.6%, the content reduction of netarsudil in the aqueous composition is significantly suppressed and the light stability is improved.

[Test Example 4] Light Stability Test Part 4

In the same way as in Test Example 3, in an attempt to obtain further stabilization, the aqueous composition comprising netarsudil was housed in a container blended with an ultraviolet scattering agent.

In other words, the test was performed in the same method as in Test Example 3 except that a container (primary package) having a shrink film blended with an ultraviolet scattering agent described below was used instead of the container blended with an ultraviolet absorbing agent.

<Container Having Shrink Film Blended with Ultraviolet Scattering Agent>

An eye drop container formed of polypropylene having a white color shrink film (heat-shrinkable film) (IWATA LABEL Co., Ltd.) kneaded with titanium oxide as an ultraviolet scattering agent in an amount of 40 to 45 % by mass wound around according to an ordinary method on a side surface of the container was used as a container. The average value of transmittance of the light of the shrink film comprising titanium oxide was 0% in 320 to 380 nm (0% on average in any of 300 to 380 nm, 270 to 380 nm, 320 to 395 nm, 300 to 395 nm, and 270 to 395 nm).

Since the shrink film is not transparent, the inside of the container was not visible from the side surface in the original state (e.g., the transmittance of the light was 2.2% on average in the visible light region (450 to 750 nm)). Therefore, slits with the width of about 5 mm were provided on the side surface of the bottom half of the container to allow the inside of the container to be visible (the part provided with the slits was in a state that could not block the light). Further, the shrink film was not wound on the bottom surface so that the inside of the container was visible from the bottom surface.

The transmittance of the light was measured at 5 nm intervals using a spectrophotometer (U-3900: Hitachi High-Technologies Corporation) after the film was set in an optical path so that light enters the film nearly vertically. The average value of the transmittance of the light was calculated as an average value of the measured transmittance of the light in a predetermined wavelength range at 5 nm intervals.

In addition, a graph showing the relationship in this container between the wavelength and transmittance (%) of the light of the range of 270 to 800 nm was shown in Fig. 2.

The results are shown in Table 5.

[Table 5]

| | Properties of package | Cumulative irradiation dose: 1.2 million lux·hr |
|---|---|---|
| Residual rate (%) | Container (primary package) formed of polypropylene having a wound shrink film comprising titanium oxide Average value of transmittance of light of 320 to 380 nm: 0% | 100 |

As shown in the result listed in Table 5, it was revealed that, by housing the aqueous composition comprising

netarsudil in a container (primary package) of which the average value of the transmittance of the light having a wavelength of from 320 to 380 nm is 0%, the content reduction of netarsudil in the aqueous composition is almost completely suppressed and the light stability is improved.

[Test Example 5] Light Stability Test Part 5

In the same way as Test Example 3, in an attempt to obtain further stabilization, the aqueous composition comprising netarsudil was housed in a container blended with an ultraviolet absorbing agent.

In other words, the aqueous composition having the formulation shown in Table 1 was prepared, and the prepared composition was housed in an eye drop container formed of polypropylene (PP) not comprising the ultraviolet absorbing agent (the container used in Test Example 1) or an eye drop container formed of polyethylene terephthalate (PET) not comprising the ultraviolet absorbing agent, as a primary package. Then, the housed composition was further housed in a transparent bag for eye drop instillation (secondary package) formed of polyethylene blended with an ultraviolet absorbing agent described below to obtain a pharmaceutical preparation.

Using the obtained pharmaceutical preparation, tests were performed in the same method as in Test Example 3.

<Transparent Bag for Eye Drop Instillation Formed of Polyethylene Blended with Ultraviolet Absorbing Agent>

The average value of the transmittance of the light of the bag for eye drop instillation was 2.6% in 320 to 380 nm (2.0% on average in 300 to 380 nm; 1.47% on average in 270 to 380 nm; 4.8% on average in 320 to 395 nm; 3.8% on average in 300 to 395 nm; and 3.0% on average in 270 to 395 nm).

The container appearance was white translucent and the content inside was visible (for example, the transmittance of the light in the visible light region (450 to 750 nm) was more than 45% in all wavelengths and 70.5% on average.

The transmittance of the light was measured at 5 nm intervals using a spectrophotometer (U-3900: Hitachi High-Technologies Corporation) after the bag was cut into a film piece and set in an optical path so that light enters the piece nearly vertically. The average value of the transmittance of the light was calculated as an average value of the measured transmittance of the light in a predetermined wavelength range at 5 nm intervals.

In addition, a graph showing the relationship in the bag for eye drop instillation between the wavelength and transmittance (%) of the light of the range of 270 to 800 nm was shown in Fig. 3.

The results are shown in Table 6.

[Table 6]

| | Properties of package | | Cumulative irradiation dose: 1.2 million lux·hr |
|---|---|---|---|
| | Secondary package | Primary package | |
| Residual rate (%) | Bag for eye drop instillation formed of polyethylene blended with ultraviolet absorbing agent Average value of transmittance of light of 320 to 380 nm: 2.6% | Container formed of PP | 98.5 |
| | | Container formed of PET | 99.0 |

As shown in the results in Table 6, it was revealed that, by housing the aqueous composition comprising netarsudil in a bag (secondary package) of which the average value of the transmittance of a light of 320 to 380 nm is 2.6%, regardless of the material of the primary package, the content reduction of netarsudil in the aqueous composition is significantly suppressed and the light stability is improved.

As shown in the results indicated in Test Examples 3 to 5 above, it was confirmed that, by housing the aqueous composition comprising the compound represented by the formula (1) exemplified by netarsudil or a salt thereof or a solvate thereof in a package which blocks a light having a wavelength of from 320 to 380 nm, the light stability of the compound represented by the formula (1) in the aqueous composition is improved.

[Test Example 6] Heat Stability Test

The heat stability of netarsudil in the aqueous composition was assessed by examining the presence or absence of content reduction of netarsudil after stored under high temperature for a certain period.

In other words, the aqueous composition having the formulation shown in Table 7 was prepared with an ordinary method, and then the obtained composition was housed in a container formed of polyethylene terephthalate (PET), low-density polyethylene (LDPE), high density polyethylene (HDPE), polypropylene (PP) or glass to obtain pharmaceutical preparations.

Each of the obtained pharmaceutical preparations was stored at 80°C for a week. To examine the presence or absence of content reduction of netarsudil after storage, the concentrations of netarsudil in the aqueous composition before and after storage were calculated by the same method as in Test Example 1.

Then, based on the calculated concentration of netarsudil in the aqueous composition, the residual rate (%) of netarsudil was assessed using the following expression:

Residual rate (%) = [(Concentration of netarsudil in the aqueous composition after storage)/(Concentration of netarsudil in the aqueous composition before storage)] × 100

The results are shown in Table 8.

[Table 7]

| Component | Amount (per 100 mL) |
|---|---|
| Netarsudil dimesylate | 0.02848 g (0.02 g in terms of a free form) |
| Boric acid | 0.05 g |
| Sodium hydroxide | q.s. (pH 5.0) |
| Purified water | Balance |

[Table 8]

| | Material of container | After storage at 80°C for a week |
|---|---|---|
| Residual rate (%) | PET | 75.1 |
| | LDPE | 72.1 |
| | HDPE | 71.4 |
| | PP | 70.7 |
| | Glass | 47.5 |

As shown in the results indicated in Table 8, when the aqueous composition comprising netarsudil was housed in the container formed of polyethylene terephthalate (PET), formed of polyethylene (LDPE, HDPE), or formed of polypropylene (PP), the content reduction due to storage under high temperature was suppressed relative to the case housed in a container formed of glass. In particular, when the aqueous composition was housed in a container formed of polyethylene terephthalate (PET) or polyethylene (LDPE, HDPE), the degree of suppression of the content reduction was significant.

[Preparation Examples 1 to 36]

Pharmaceutical preparations of Preparation Examples 1 to 36 can be prepared by preparing aqueous compositions each comprising the components in amounts ((g) per 100 mL of the aqueous composition) shown in Tables 9 to 17 with an ordinary method, and housing the prepared aqueous composition each in an eye drop container (primary package) formed of polypropylene kneaded with a benzotriazole-based ultraviolet absorbing agent which was used in Test Example 3.

[Table 9]

| | Preparation Examples | | | |
|---|---|---|---|---|
| | 1 | 2 | 3 | 4 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Sodium edetate | | 0.01 | 0.01 | |
| D-mannitol | 4.7 | | | |
| Glycerin | | 2.5 | | |
| Propylene glycol | | | 1.7 | |
| Xylitol | | | 0.7 | |
| Macrogol 6000 | | | | 1 |
| Macrogol 4000 | | | | 4 |
| Sorbitol | | | | |
| Trometamol | | | | |
| Benzalkonium chloride | 0.015 | 0.03 | 0.05 | |
| Benzododecinium bromide | | | | 0.01 |
| Benzethonium chloride | | | | 0.01 |
| Sodium chloride | | 0.1 | | |
| Potassium chloride | | | | 0.2 |
| Calcium chloride | | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 10]

| | Preparation Examples | | | |
|---|---|---|---|---|
| | 5 | 6 | 7 | 8 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Sodium edetate | | 0.01 | 0.01 | |
| D-mannitol | | | 1 | 3 |
| Glycerin | | | 3 | |
| Propylene glycol | | | | 1 |
| Xylitol | | | | |
| Macrogol 6000 | | | | |
| Macrogol 4000 | | | | |
| Sorbitol | 0.86 | | | |
| Trometamol | | 20 | | |
| Benzalkonium chloride | 0.015 | 0.03 | 0.05 | |
| Benzododecinium bromide | | | | 0.01 |
| Benzethonium chloride | | | | 0.01 |
| Sodium chloride | | | 0.05 | |
| Potassium chloride | | | 0.05 | |
| Calcium chloride | 0.1 | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 11]

| | Preparation Examples | | | |
|---|---|---|---|---|
| | 9 | 10 | 11 | 12 |
| Netarsudil dimesylate | 0.01424 g (0.01 g in terms of a free form | 0.05696 g (0.04 g in terms of a free form) | 0.02848 g (0.02 g in terms of a free form) | 0.02848 g (0.02 g in terms of a free form) |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Sodium edetate | | 0.01 | 0.01 | |
| D-mannitol | 2 | 2 | | 2 |
| Glycerin | 2 | 2 | 1 | |
| Propylene glycol | | | | 1.5 |
| Xylitol | 0.3 | | | |
| Macrogol 6000 | 1 | | | |
| Macrogol 4000 | | 2 | | |
| Sorbitol | | | 0.5 | |
| Trometamol | | | 10 | |
| Benzalkonium chloride | 0.015 | 0.03 | 0.05 | |
| Benzododecinium bromide | | | | 0.01 |
| Benzethonium chloride | | | | 0.01 |
| Sodium chloride | | 0.1 | | |
| Potassium chloride | | | | 0.2 |
| Calcium chloride | | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 12]

| | Preparation Examples | | | |
|---|---|---|---|---|
| | 13 | 14 | 15 | 16 |
| Netarsudil dimesylate | 0.02848 g (0.02 g in terms of a free form) | 0.02848 g (0.02 g in terms of a free form) | 0.01424 g (0.01 g in terms of a free form) | 0.05696 g (0.04 g in terms of a free form) |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Sodium edetate | | 0.01 | 0.01 | |
| D-mannitol | 3 | 1 | 1 | 2 |
| Glycerin | 1 | | 1 | |
| Propylene glycol | | | | 1 |
| Xylitol | | | | |
| Macrogol 6000 | | 1 | | |
| Macrogol 4000 | | 2 | | |
| Sorbitol | | | | 0.5 |
| Trometamol | | | 15 | |
| Benzalkonium chloride | 0.015 | 0.03 | 0.05 | |
| Benzododecinium bromide | | | | 0.01 |
| Benzethonium chloride | | | | 0.01 |
| Sodium chloride | | | 0.05 | |
| Potassium chloride | | | 0.05 | |
| Calcium chloride | 0.1 | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 13]

| | Preparation Examples | | | |
|---|---|---|---|---|
| | 17 | 18 | 19 | 20 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Sodium edetate | 0.01 | 0.01 | 0.01 | 0.01 |
| D-mannitol | 4.7 | | | |
| Glycerin | | 2.5 | | |
| Propylene glycol | | | 1.7 | |
| Xylitol | | | 0.7 | |
| Macrogol 400 | 2.5 | 1 | | 4 |
| Macrogol 4000 | | | | 1 |
| Sorbitol | | | | |
| Trometamol | | | | |
| Benzalkonium chloride | 0.2 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |

(continued)

| | Preparation Examples | | | |
|---|---|---|---|---|
| | 17 | 18 | 19 | 20 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Purified water | Total Amount 100 mL | Total Amount 100 mL | Total Amount 100 mL | Total Amount 100 mL |
| pH | 5.0 | 5.0 | 5.0 | 5.0 |

[Table 14]

| | Preparation Examples | | | |
|---|---|---|---|---|
| | 21 | 22 | 23 | 24 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Sodium edetate | | 0.01 | 0.01 | |
| D-mannitol | | | 1 | 3 |
| Glycerin | | | 3 | |
| Propylene glycol | | | | 1 |
| Xylitol | | | | |
| Macrogol 400 | | | | |
| Macrogol 4000 | | | | |
| Sorbitol | 0.86 | | | |
| Trometamol | | 20 | | |
| Benzalkonium chloride | 0.015 | 0.03 | 0.05 | |
| Benzododecinium bromide | | | | 0.01 |
| Benzethonium chloride | | | | 0.01 |

(continued)

| | Preparation Examples | | | |
|---|---|---|---|---|
| | 21 | 22 | 23 | 24 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 6.0 | 5.5 | 6.0 | 5.5 |

[Table 15]

| | Preparation Examples | | | |
|---|---|---|---|---|
| | 25 | 26 | 27 | 28 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Sodium edetate | | 0.01 | 0.01 | |
| D-mannitol | 2 | 2 | | 2 |
| Glycerin | 2 | 2 | 1 | |
| Propylene glycol | | | | 1.5 |
| Xylitol | 0.3 | | | |

(continued)

| | Preparation Examples | | | |
|---|---|---|---|---|
| | 25 | 26 | 27 | 28 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Macrogol 400 | | 2 | | |
| Macrogol 4000 | | 1 | | |
| Sorbitol | | | 0.5 | |
| Trometamol | | | 10 | |
| Benzalkonium chloride | 0.015 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 7.0 | 6.0 | 7.0 | 6.0 |

[Table 16]

| | Preparation Examples | | | |
|---|---|---|---|---|
| | 29 | 30 | 31 | 32 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | | | | |
| Borax | | | | |
| Sodium dihydrogen phosphate | | | | |
| Disodium hydrogen phosphate | | | | |
| Citric acid | 0.12 | 0.05 | | |

(continued)

| | Preparation Examples | | | |
|---|---|---|---|---|
| | 29 | 30 | 31 | 32 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Sodium citrate | 0.4 | 0.03 | | |
| Acetic acid | | | 0.001 | |
| Sodium acetate | | | 0.2 | 0.1 |
| Sodium edetate | | 0.01 | 0.01 | |
| D-mannitol | 3 | 1 | 1 | 2 |
| Glycerin | 1 | | 1 | |
| Propylene glycol | | | | 1 |
| Xylitol | | | | |
| Macrogol 400 | | 2 | | |
| Macrogol 4000 | | 1 | | |
| Sorbitol | | | | 0.5 |
| Trometamol | | | 15 | |
| Benzalkonium chloride | 0.015 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 6.0 | 5.5 | 6.0 | 5.5 |

[Table 17]

| | Preparation Examples | | | |
|---|---|---|---|---|
| | 33 | 34 | 35 | 36 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Timolol maleate | 6.83 mg (5.0 mg in terms of a free form) | 6.83 mg (5.0 mg in terms of a free form) | 6.83 mg (5.0 mg in terms of a free form) | 6.83 mg (5.0 mg in terms of a free form) |
| Boric acid | 0.04 | 0.05 | | |

(continued)

| | Preparation Examples | | | |
|---|---|---|---|---|
| | 33 | 34 | 35 | 36 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Timolol maleate | 6.83 mg (5.0 mg in terms of a free form) | 6.83 mg (5.0 mg in terms of a free form) | 6.83 mg (5.0 mg in terms of a free form) | 6.83 mg (5.0 mg in terms of a free form) |
| Borax | 0.01 | | | |
| Sodium dihydrogen phosphate | | 0.031 | | |
| Disodium hydrogen phosphate | | 0.07 | | |
| Citric acid | | | 0.05 | |
| Sodium citrate | | | 0.03 | |
| Acetic acid | | | | 0.001 |
| Sodium acetate | | | | 0.2 |
| Sodium edetate | 0.01 | 0.01 | 0.01 | 0.01 |
| D-mannitol | 4.7 | | | |
| Glycerin | | 2.5 | | |
| Propylene glycol | | | 1.7 | |
| Xylitol | | | 0.7 | |
| Macrogol 400 | | 1 | | |
| Macrogol 4000 | | | 1 | |
| Sorbitol | | | | 3 |
| Trometamol | | | | 15 |
| Benzalkonium chloride | 0.015 | 0.03 | 0.05 | |
| Benzododecinium bromide | | | | 0.01 |
| Benzethonium chloride | | | | 0.01 |
| Methyl cellulose | 0.5 | 2 | | |
| Gellan gum | | | 0.2 | 0.6 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 6.0 | 7.0 | 6.0 |

[Preparation Examples 37 to 72]

Pharmaceutical preparations of Preparation Examples 37 to 72 can be prepared in the same way as in Preparation Examples 1 to 36 by using the container as used in Test Example 3 except that the material of the container was polypropylene instead of polyethylene.

[Preparation Examples 73 to 108]

Pharmaceutical preparations of Preparation Examples 73 to 108 can be prepared in the same way as in Preparation Examples 1 to 36 by using the eye drop container formed of polypropylene having a shrink film comprising titanium oxide (primary package) wound around on a side surface of the container which was used in Test Example 4 instead of the eye drop container formed of polypropylene kneaded with a benzotriazole-based ultraviolet absorbing agent.

[Preparation Examples 109 to 144]

Pharmaceutical preparations of Preparation Examples 109 to 144 can be prepared in the same way as in Preparation Examples 1 to 36 by using, as the container, an eye drop container formed of polyethylene terephthalate instead of the eye drop container formed of polypropylene kneaded with a benzotriazole-based ultraviolet absorbing agent, and further housing the composition housed in the container in a box formed of paper (secondary package).

[Preparation Examples 145 to 288]

Pharmaceutical preparations of Preparation Examples 145 to 288 can be prepared with an ordinary method by using 0.5 g of verosudil monohydrochloride in a free form instead of netarsudil dimesylate in the Preparation Examples 1 to 144.

[Preparation Examples 289 to 432]

Pharmaceutical preparations of Preparation Examples 289 to 432 can be prepared with an ordinary method by using 0.7 g of verosudil monohydrochloride in a free form instead of netarsudil dimesylate in the Preparation Examples 1 to 144.

[Preparation Examples 433 to 576]

Pharmaceutical preparations of Preparation Examples 433 to 576 can be prepared with an ordinary method by using 0.02 g of a compound represented by the formula (8) instead of netarsudil dimesylate in the Preparation Examples 1 to 144.

Industrial Applicability

According to the present invention, a pharmaceutical preparation having excellent stability can be provided, and it can be suitably used in the pharmaceutical industry and the like.

## Claims

1. A pharmaceutical preparation comprising:
an aqueous composition comprising a compound represented by the following formula (1)

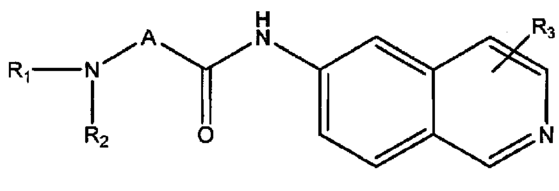

(1)

(in the formula, $R_1$ and $R_2$ each independently represent a hydrogen atom or a $C_1$-$C_4$ alkyl group,
$R_3$ represents a hydrogen atom or a hydroxy group, and
"A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and
the formula (1) also includes a tautomer thereof) or a salt thereof or a solvate thereof, the aqueous composition being housed in a package which blocks a light having a wavelength of from 320 to 380 nm.

**2.** The pharmaceutical preparation according to claim 1, wherein the compound represented by the formula (1) is a compound represented by the following formula (2), (5), (5') or (8) :

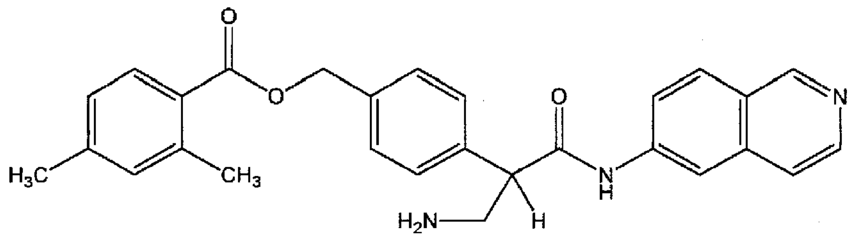

(2) ;

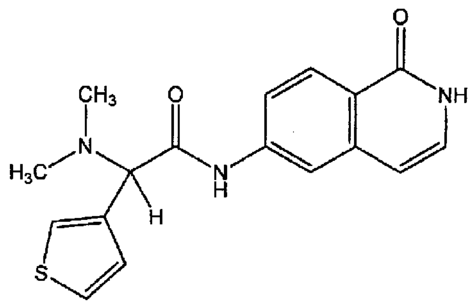

(5) ;

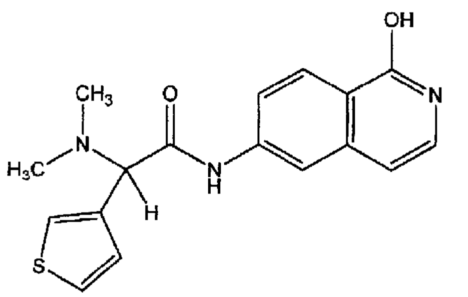

(5') ;

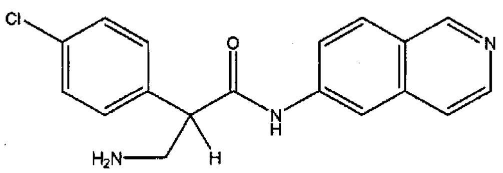

(8)

.

**3.** The pharmaceutical preparation according to claim 1, wherein the compound represented by the formula (1) is a compound represented by the following formula (3):

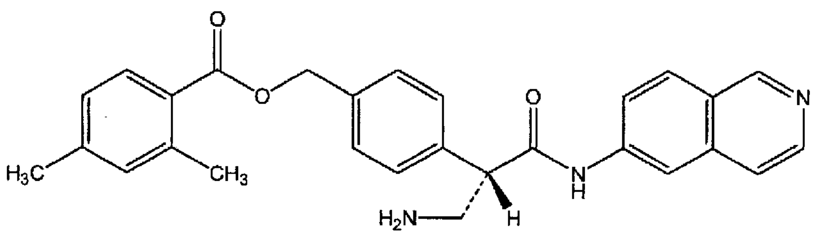

(3)

.

**4.** The pharmaceutical preparation according to any one of claims 1 to 3, wherein a residual rate of the compound represented by the formula (1) in the aqueous composition after irradiation is 80% or more, when the aqueous

composition is irradiated with a light so that a cumulative irradiation dose at a temperature of 25±5°C is 1.2 million lux·hr using a D65 fluorescent lamp as a light source.

5. The pharmaceutical preparation according to any one of claims 1 to 4, wherein inside of the package is visible.

6. The pharmaceutical preparation according to any one of claims 1 to 5, wherein an average value of transmittance of a light having a wavelength of from 450 to 750 nm of the package is 30 % or more.

7. The pharmaceutical preparation according to any one of claims 1 to 6, wherein the package includes, as a primary package, a container comprising a substance which interferes with transmission of ultraviolet light.

8. The pharmaceutical preparation according to any one of claims 1 to 7, wherein the package includes, as a primary package, a container having a member comprising a substance which interferes with transmission of ultraviolet light.

9. The pharmaceutical preparation according to any one of claims 1 to 8, wherein the package includes, as a secondary package, a bag comprising a substance which interferes with transmission of ultraviolet light.

10. A method for improving light stability of a compound represented by the following formula (1):

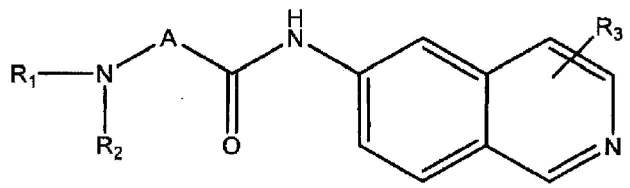

(1)

(in the formula, $R_1$ and $R_2$ each independently represent a hydrogen atom or a $C_1$-$C_4$ alkyl group, $R_3$ represents a hydrogen atom or a hydroxy group, and "A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and the formula (1) also includes a tautomer thereof) or a salt thereof or a solvate thereof in an aqueous composition, comprising a step of housing the aqueous composition comprising the compound represented by the formula (1) or a salt thereof or a solvate thereof in a package which blocks a light having a wavelength of from 320 to 380 nm.

11. The method according to claim 10, wherein the compound represented by the formula (1) is a compound represented by the formula (2), (5), (5') or (8):

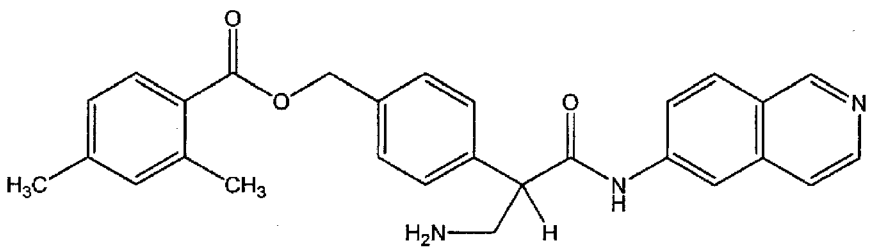

(2) ;

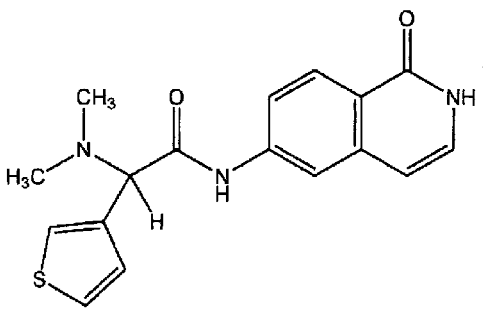

(5) ;

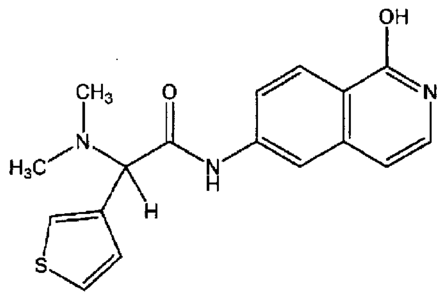

(5') ;

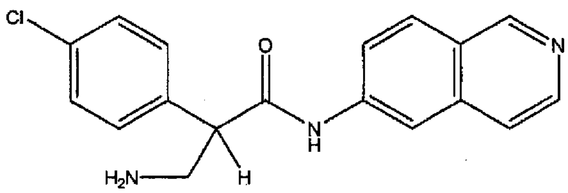

(8)

.

12. The method according to claim 10, wherein the compound represented by the formula (1) is a compound represented by the following formula (3):

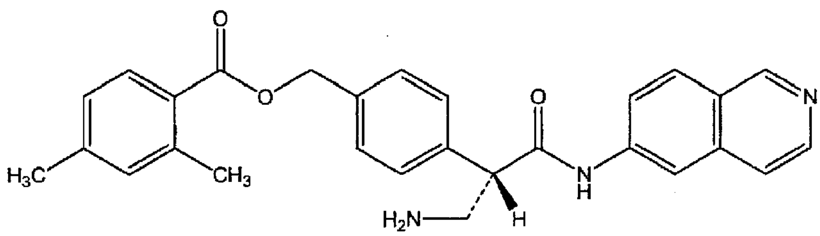

(3)

.

13. The method according to any one of claims 10 to 12, wherein, a residual rate of the compound represented by the formula (1) in the aqueous composition after irradiation is 80% or more, when the aqueous composition is irradiated with a light so that a cumulative irradiation dose at a temperature of 25±5°C is 1.2 million lux·hr using a D65 fluorescent lamp as a light source.

14. The method according to any one of claims 10 to 13, wherein the package includes, as a primary package, a container comprising a substance which interferes with transmission of ultraviolet light.

15. The method according to any one of claims 10 to 14, wherein the package includes, as a primary package, a container having a member comprising a substance which interferes with transmission of ultraviolet light.

16. The method according to any one of claims 10 to 15, wherein the package includes, as a secondary package, a bag comprising a substance which interferes with transmission of ultraviolet light.

[Figure 1]

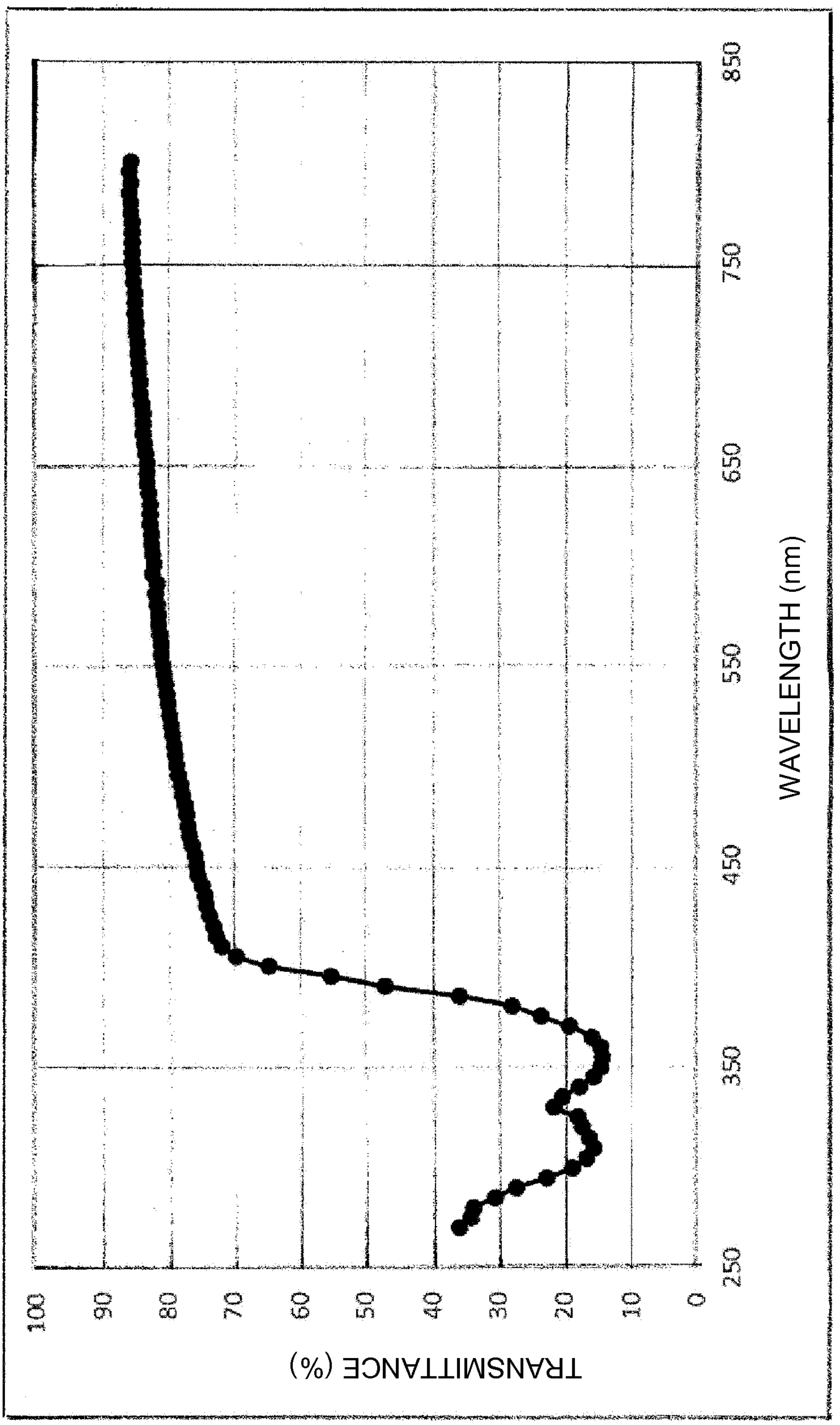
TRANSMITTANCE (%)
100
90
80
70
60
50
40
30
20
10
0
250
350
450
550
650
750
850
WAVELENGTH (nm)

[Figure 2]

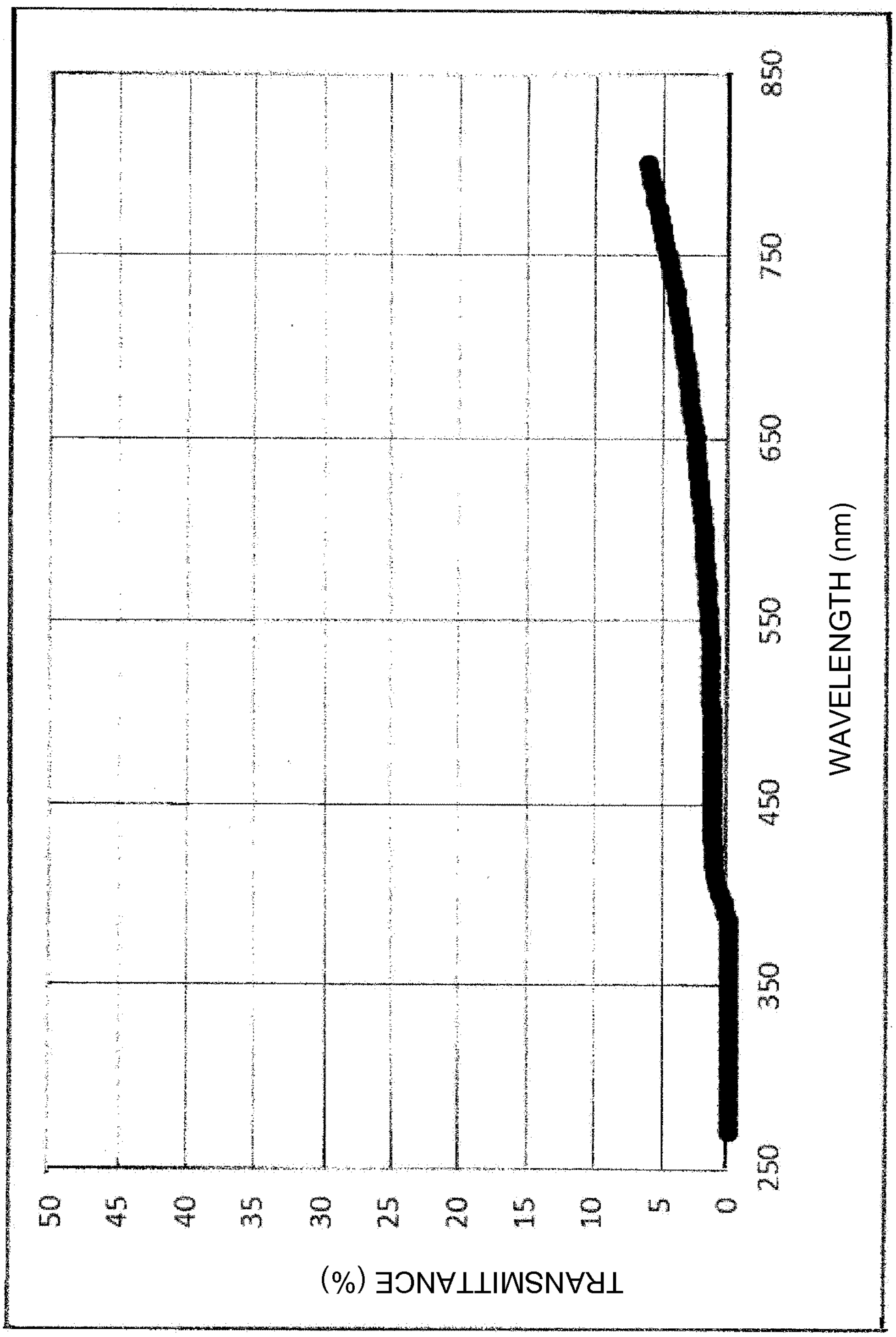
TRANSMITTANCE (%)
50
45
40
35
30
25
20
15
10
5
0
250
350
450
550
650
750
850
WAVELENGTH (nm)

[Figure 3]

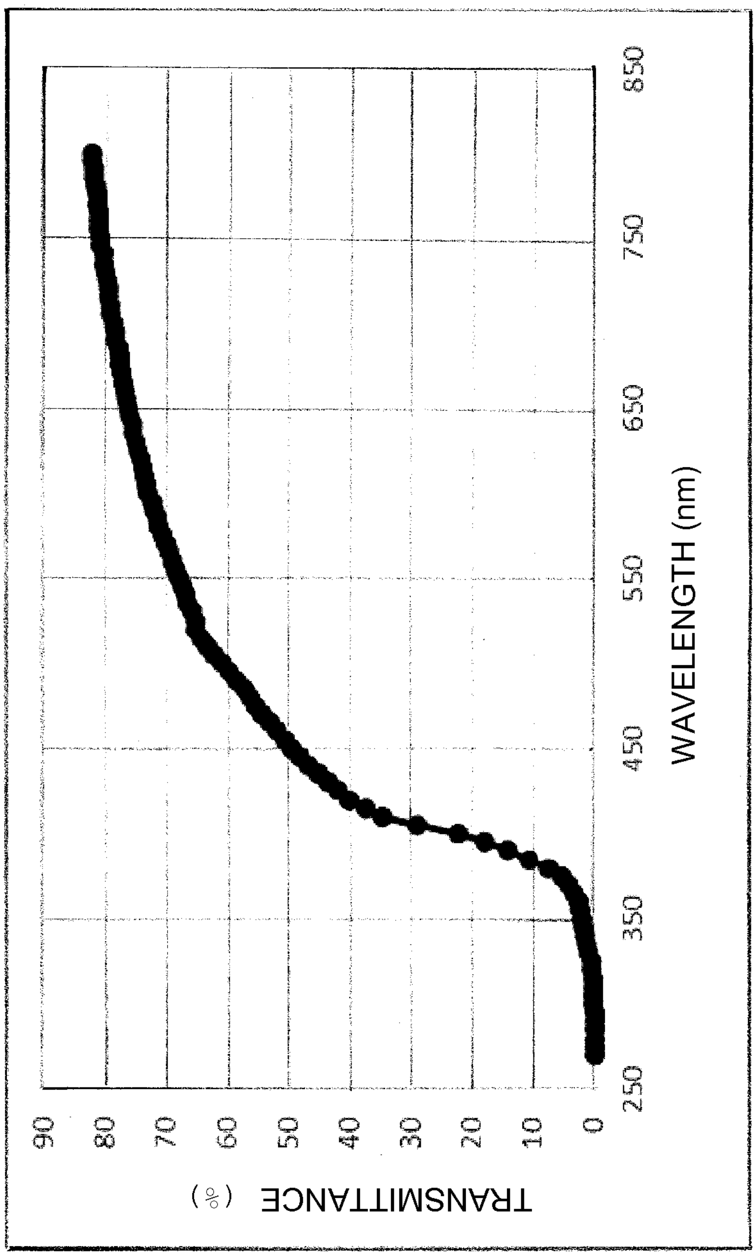
TRANSMITTANCE (%)
0
10
20
30
40
50
60
70
80
90
WAVELENGTH (nm)
250
350
450
550
650
750
850

## INTERNATIONAL SEARCH REPORT

International application No.
PCT/JP2018/007581

**A. CLASSIFICATION OF SUBJECT MATTER**

Int.Cl. A61K31/472(2006.01)i, A61J1/05(2006.01)i, A61K9/08(2006.01)i, A61K31/4725(2006.01)i, A61K47/04(2006.01)i, A61K47/10(2006.01)i, A61P27/02(2006.01)i, A61P27/06(2006.01)i, B65D1/00(2006.01)i, B65D65/20(2006.01)i

According to International Patent Classification (IPC) or to both national classification and IPC

**B. FIELDS SEARCHED**

Minimum documentation searched (classification system followed by classification symbols)

Int.Cl. A61K31/472, A61J1/05, A61K9/08, A61K31/4725, A61K47/04, A61K47/10, A61P27/02, A61P27/06, B65D1/00, B65D65/20

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

CAplus/REGlSTY/MEDLlNE/EMUASE/BIOSIS (STN), WPI, JSTPlus/JMEDPlus/JST7580 (JDreamIII), Science Direct, PubMed

**C. DOCUMENTS CONSIDERED TO BE RELEVANT**

| Category* | Citation of document, with indication, where appropriate, of the relevant passages | Relevant to claim No. |
|---|---|---|
| Y | JP 2016-515520 A (AERIE PHARMACEUTICALS, INC.) 30 May 2016, claims, paragraphs [0199], [0200], examples 1-6 & WO 2014/144781 A1, claims, paragraphs [0191], [0192], examples 1-6 & EP 2976080 A1 & US 2014/0275160 A1 | 1-16 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 March 2018 (22.03.2018) | 03 April 2018 (03.04.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/007581

C (Continuation). DOCUMENTS CONSIDERED TO BE RELEVANT

| Category* | Citation of document, with indication, where appropriate, of the relevant passages | Relevant to claim No. |
|---|---|---|
| Y | ELLIS, Eydie Miller et al., "Ocular hypotensive effect of the novel EP3/FP agonist ONO-9054 versus Xalatan: results of a 28-day, double-masked, randomised study", British Journal of Ophthalmology, 20 September 2016, vol. 101, pp. 796-800, Study design, ISSN:1468-2079 | 1-16 |
| A | WO 2016/047720 A1 (KOWA CO., LTD.) 31 March 2016 & US 2017/0266080 A1 & EP 3199162 A1 | 1-16 |
| A | WO 2005/087237 A1 (ASAHI KASEI PHARMA CORPORATION) 22 September 2005 & US 2008/0234483 A1 & EP 1726306 A1 | 1-16 |
| A | JP 2016-138085 A (ROHTO PHARMACEUTICAL CO., LTD.) 04 August 2016 (Family: none) | 1-16 |
| A | LEVY, Brian et al., "Ocular hypotensive safety and systemic absorption of AR-13324 ophthalmic solution in normal volunteers", American Journal of Ophthalmology, May 2015, vol. 159, no. 5, pp. 980-985.el, ISSN:0002-9394 | 1-16 |
| A | REN, Ruiyi et al., "Netarsudil increases outflow facility in human eyes through multiple mechanisms", Investigative Ophthalmology and Visual Science, November 2016, vol. 57, no. 14, pp. 6197-6209, ISSN:1552-5783 | 1-16 |
| P, X | RHOPRESSA™ (netarsudil ophthalmic solution) Label, NDA 208254, Reference ID:4194833, December 2017, pp. 4-10 | 1-16 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012525386 A
- WO 2009091898 A
- JP 2016515520 A


### Non-patent literature cited in the description


- **BACHARACH et al.** *Ophthalmology,* 2015, vol. 122 (2), 302-7
- **STURDIVANT JM. et al.** *Bioorg Med Chem Lett.,* 2016, vol. 26 (10), 2475-80
- **WILLIAMS RD. et al.** *Am. J. Ophthalmol.,* 2011, vol. 152 (5), 834-41
- Pharmaceutical Excipients Dictionary. Yakuji Nippo, 2016
- Japanese Pharmacopoeia